# EUROPEAN PATENT SPECIFICATION

(11) **EP 2 700 713 B1**
(45) Date of publication and mention of the grant of the patent: **13.07.2016**
(21) Application number: 12181138.4
(22) Date of filing: 21.08.2012
(51) Int. Cl.: C12N 15/79, C12N 15/10

(54) **Screening and enrichment system for protein expression in eukaryotic cells using a tricistronic expression cassette**
Untersuchungs- und Anreicherungssystem für die Proteinexpression in eukaryotischen Zellen unter Verwendung einer trizistronischen Expressionskassette
Système d'enrichissement et de criblage pour l'expression de protéines dans des cellules eucaryotes à l'aide d'une cassette d'expression tricistronique

(43) Date of publication of application: 26.02.2014
(73) Proprietor: Miltenyi Biotec GmbH, 51429 Bergisch Gladbach (DE)
(72) Inventor: Levin, Anne, 51063 Köln (DE); Eckardt, Nicole Susann, Dr., 51429 Bergisch Gladbach (DE); Bürger, Iris, 51503 Rösrath (DE)

(56) References cited:
- WO-A1-98/11241
- DE FELIPE P ET AL: "Tricistronic and tetracistronic retroviral vectors for gene transfer", HUMAN GENE THERAPY, MARY ANN LIEBERT, NEW YORK ,NY, US, vol. 11, no. 13, 1 September 2000 (2000-09-01), pages 1921-1931, XP002380365, ISSN: 1043-0342, DOI: 10.1089/10430340050129530
- FELIPE DE P ET AL: "Use of the 2A sequence from foot-and-mouth disease virus in the generation of retroviral vectors for gene therapy", GENE THERAPY, MACMILLAN PRESS LTD., BASINGSTOKE, GB, vol. 6, no. 2, 1 February 1999 (1999-02-01), pages 198-208, XP002251976, ISSN: 0969-7128, DOI: 10.1038/SJ.GT.3300811
- CHINNASAMY DHANALAKSHMI ET AL: "Multicistronic lentiviral vectors containing the FMDV 2A cleavage factor demonstrate robust expression of encoded genes at limiting MOI", VIROLOGY JOURNAL, BIOMED CENTRAL, LONDON, GB, vol. 3, no. 1, 15 March 2006 (2006-03-15), page 14, XP021019318, ISSN: 1743-422X, DOI: 10.1186/1743-422X-3-14
- MILSOM M D ET AL: "Enhanced in vivo selection of bone marrow cells by retroviral-mediated coexpression of mutant O<6>-methylguanine-DNA-methyltransferase and HOXB4", MOLECULAR THERAPY, ACADEMIC PRESS, SAN DIEGO, CA, US, vol. 10, no. 5, 1 November 2004 (2004-11-01), pages 862-873, XP004660738, ISSN: 1525-0016, DOI: 10.1016/J.YMTHE.2004.07.019
- CHINNASAMY NACHIMUTHU ET AL: "Development of novel multigene lentiviral vectors", BLOOD, AMERICAN SOCIETY OF HEMATOLOGY, US, vol. 104, no. 11, Part 2, 7 December 2004 (2004-12-07), XP009124692, ISSN: 0006-4971
- STEVEN C L HO ET AL: "IRES-mediated Tricistronic vectors for enhancing generation of high monoclonal antibody expressing CHO cell lines", JOURNAL OF BIOTECHNOLOGY, ELSEVIER SCIENCE PUBLISHERS, AMSTERDAM, NL, vol. 157, no. 1, 19 September 2011 (2011-09-19), pages 130-139, XP028395739, ISSN: 0168-1656, DOI: 10.1016/J.JBIOTEC.2011.09.023 [retrieved on 2011-10-17]
- FUSSENEGGER MARTIN ET AL: "pTRIDENT, a novel vector family for tricistronic gene expression in mammalian cells", BIOTECHNOLOGY AND BIOENGINEERING, vol. 57, no. 1, 5 January 1998 (1998-01-05), pages 1-10, XP002688887, ISSN: 0006-3592
- TRICHAS GEORGIOS ET AL: "Use of the viral 2A peptide for bicistronic expression in transgenic mice", BMC BIOLOGY, BIOMED CENTRAL, LONDON, GB, GB, vol. 6, no. 1, 15 September 2008 (2008-09-15), page 40, XP021044858, ISSN: 1741-7007, DOI: 10.1186/1741-7007-6-40

## Description

### Field of invention

The present invention relates to a screening and enrichment system for protein expression in eukaryotic cells. In particular, it relates to a method for screening cells to identify rapidly those cells expressing the gene of interest at high levels of protein.

### Background of the invention

Mammalian expression systems provide correct protein folding and appropriate post-translational modifications. However, expression in mammalian cells often results in low protein yields. Current available and commonly used expression systems based on selection with metabolic enzymes are the dihydrofolate reductase (DHFR) amplification system (Haynes J, Weissmann C. (1983) Nucleic Acids Res. 11(3): 687-706) which is based on the selection with the enzyme dihydrofolate reductase in a medium lacking hypoxanthine and thymidine, and the GS Gene Expression System^{™} (Cockett MI et al (1990) Nat Biotechnol. 1990; 8: 662 - 667) which is based on the selection with the enzyme glutamine synthetase (GS) in a glutamine-free medium. Although the DHFR selection is still one of the most frequently used approaches the major disadvantages are time-consuming amplification steps and the high cytotoxicity (mutagenicity, teratogenicity) of the DHFR-inhibitor methotrexate (MTX). The use of the drug methotrexate facilitates high copy number and therefore increased recombinant protein production, but with the costs of implications for genetic instability of the production cell lines (Kim SJ et al (1998) Biotech Bioeng. 1998; 58: 73-84). Although the GS Gene Expression System^{™} can be used without the time-consuming amplification process, similar to the DHFR selection system the permanent use of an inhibitory, toxic drug is necessary. Since Chinese hamster ovary (CHO) cells endogenously express the glutamine synthetase, the GS-inhibitor methionine sulphoximine (MSX) is required to maintain the productivity at a consistent level. Recently GS-knock out cell lines have been described (Liu PQ et al (2010) Biotech Bioeng. 2010; 106: 97-105).
Thus, in every above-mentioned case the application of inhibitory drugs is part of the method leading to expensive, time-consuming, and "safety-relevant" procedures. In addition, those expression systems do not provide an inherent method for screening of cells to identify rapidly those cells expressing the gene of interest at high levels of protein.

The CHO cell line has been shown to be proline auxotroph due to a deficiency in both available proline catabolic pathways (Valle, D et al (1973), Biochem Biophys Res Commun 53: 1130-1136). One of the crucial enzymes is the metabolic enzyme pyrroline-5-carboxylate synthetase (P5CS). Hu et al (J Biol Chem (1999), 274: 6754-62) transfected murine P5CS cDNA into a sub-clone of CHO-K1 cells known for deficiency in P5CS activity. They selected stable transfectants in medium lacking proline and assayed P5CS activity on individual clones. So far no selection and protein expression system relying on the proline auxotrophy of the CHO cells has been described.

Expression systems free of cytotoxic drugs are disclosed e.g. in US2009/0298055A1. A method of producing a protein of interest is described, the method comprising a) transfecting eukaryotic cells lacking activity of an endogenous cell viability protein with a nucleic acid construct encoding the protein of interest and the cell viability protein; and b) culturing the eukaryotic cells transfected with the nucleic acid construct under conditions such that cell viability is dependent upon normal activity of the cell protein thereby selecting for positively-transfected cells, and c) purifying the protein of interest from the positively-transfected eukaryotic cells. In some embodiments a bicistronic expression cassette is used for selection and expression. Again, those expression systems do not provide an inherent method for screening of cells to identify rapidly those cells expressing the gene of interest at high levels of protein.

Co-expression of green fluorescent protein (GFP) enables real-time analysis and sorting of transfectants by fluorescence. The use of GFP as a reporter molecule to monitor gene transfer has been described in various publications. Chalfie et al. in U.S. Patent No. 5,491,084 describe a method of selecting cells expressing a protein of interest that involves co-transfecting cells with one deoxyribonucleic acid (DNA) molecule containing a sequence encoding a protein of interest, and a second DNA molecule, which encodes GFP, then selecting cells, which express GFP.
Mosser et al., Biotechnique 22: 150-154 (1997) describe the use of a plasmid containing a bicistronic expression cassette encoding GFP and a target gene, in a method of screening and selection of cells expressing inducible products. The target gene was linked to a controllable promoter. The plasmid incorporates a viral internal ribosome entry site (IRES) element to make it possible to express a bicistronic mRNA encoding both the GFP and a protein of interest. This plasmid described by Mosser does not contain any selectable gene; the selectable gene is provided in a separate plasmid which is transfected sequentially or co-transfected with the GFP/target gene-encoding plasmid.
US7,977,097B1 discloses a method of screening multiply transfected cells to identify those cells expressing at least two peptides or proteins of interest. The method comprising: 1. Simultaneously or sequentially transfecting a cell with at least two different bicistronic expression cassettes in which the gene of interest is linked via a IRES to a fluorescent marker gene. Each marker gene is different. 2. Providing conditions in which expression of the genes will occur. 3. Identifying cells expressing proteins by detecting the different fluorescent signals using fluorescence activated cell sorting (FACS^{™}). Both genes encoding the reporter protein and the therapeutic protein are on a bicistronic vector resulting in an independent translation although they are transcribed in the same messenger ribonucleic acid (mRNA). Since they each arise from a common mRNA, the reporter protein's expression level accurately predicts the relative expression level of the therapeutic protein for each clone resulting in the opportunity for reliable screening of cells producing high levels of therapeutic proteins. Again, US7,977,097 does not contain any selectable gene.
GFP has been successfully fused to other drug resistant gene products (see, e.g., Bennett et al., Biotechniques 24: 478-482 (1998); Primig et al., Gene 215: 181-189 (1998)). Bennett et al., describe a GFP fused to a zeocin^{™} resistance gene (ZeoR) to generate a bifunctional selectable marker for identification and selection of transfected mammalian cells. One major disadvantage of these bifunctional selectable markers is the co-localisation of the GFP with the zeocin resistance protein, leading to a cytoplasmic distribution of GFP. Therefore these bifunctional selectable markers cannot be used for methods like magnetic cell sorting technologies (e.g. MACS^{®}) where the reporter protein needs to be expressed on the cell surface.

WO9811241A1 discloses a mammalian expression system for the production of recombinant heteromeric proteins, preferably antibodies by means of oligocistronic expression vectors, which are under the control of a strong promoter/enhancer unit, a selection marker gene and at least two IRES elements. Again, this expression system does not provide an inherent method for screening of cells to identify rapidly those cells expressing the gene of interest at high levels of protein.

EP1196566B1 discloses vectors that allow a method of identifying and selecting for stable eukaryotic cells expressing high levels of a desired product by means of a polynucleotide comprising the following three components: a) an amplifiable selectable gene; b) a green fluorescent protein (GFP) gene; and c) at least one cloning site for insertion of a selected sequence encoding a desired product, wherein the selected sequence is operably linked to either the amplifiable selectable gene or to the GFP gene, and to a promoter. The polynucleotide comprises two transcription units and a promoter in each unit. The disadvantage of this method is that two independent transcription units each having its own promoter are necessary. This yields in a poor correlation of expression levels among the genes of the different transcription units. This complicates the screening of cells to identify rapidly those cells expressing the gene of interest at high levels of protein.
De Felipe (1999, GENE THERAPY, vol. 6, no. 2: 198-208) and De Felipe et al (2000, HUMAN GENE THERAPY, vol. 11, no. 13: 1921-1931) disclosed vectors which combined the autocleavage sequence from FMDV 2A and IRES elements (from EMCV and avian reticuloendotheliosis virus type A). It was showed that neither the titer nor the expression of any of the transgenes were seriously affected in these complex retroviral vectors. Chinnasamy et al (2006, VIROLOGY JOURNAL, vol. 3:14) developed multigene lentiviral vectors, incorporating 2A and IRES sequences that efficiently mediated the co-expression of two or three transgenes in multiple cell types. These multicistronic vectors are useful for various basic laboratory studies and gene therapy applications.
In Milsom et al (2004, MOLECULAR THERAPY, vol. 10, no. 5: 862-873) the authors used the combination of the FMDV 2a self-processing moiety and the EMCV IRES to demonstrate the production of a tricistronic retroviral vector that is able to express three different proteins efficiently while maintaining a titer that is more than sufficient for the transduction of repopulating BMC. Making use of both *in vitro* and *in vivo* assays, they additionally showed that each of the encoded proteins possesses normal biological activity.
Chinnasamy et al (2004, BLOOD, vol. 104, no. 11, Part 2, abstract) discussed in general the use of FMDV 2A and IRES sequences in bi- and tricistronic lentiviral vectors. The observation was made that the multigene vectors produced high titer viral particles and were able to simultaneously express two or three transgenes in transduced cells. However, the expression of EGFP from monocistronic vector as measured by mean fluorescence intensity was 3-8 times and 10- 20 times higher than that of bicistronic and tricistronic vectors respectively. Notably expression of second gene encoded by the bicistronic vector containing FMDV 2A was 2-3 fold higher than that of IRES-based vector. Expression of MGMT in monocistronic and bicistronic constructs was also 4 to 20 times higher than tricistronic vectors.

Thus, there is a need to improve the system for a fast and stable expression of a gene of interest for the production of small to medium amounts of recombinant proteins, which, in addition, is applicable to fast screening and enrichment technologies.

### Summary of the invention

Surprisingly, the inventors found that a polynucleotide comprising a tricistronic expression cassette for selection and expression in eukaryotic cells comprising a) a promoter, b) a gene of interest (GOI), c) a reporter gene, d) a selection marker gene, e) an IRES element, and f) a 2A element, wherein the order in 5' to 3'direction within said tricistronic expression cassette is: promoter - GOI - IRES element operably linked to the reporter gene or the selection marker gene - 2A element operably linked to the reporter gene or the selection marker gene, improves the method and system for a fast and stable expression of a gene of interest for the production of small to medium amounts of recombinant proteins, which, in addition, is applicable to fast screening and enrichment technologies.

All three genes encoding the protein of interest, the reporter protein and the selection marker protein are on a polynucleotide comprising a tricistronic expression cassette and are under the transcriptional control of the same promoter and are transcribed in the same mRNA but are translated into three separate, independent proteins due to the presence of an IRES element and a 2A element. Although there are three independent proteins the expression occurs in a coupled mechanism due to the presence of said IRES element and said 2A element resulting in controlled but differing levels of proteins. The interaction of the strength of the promoter, the strength of the IRES element and the order of genes within the tricistronic expression cassette decide on the level of expression of each of the three proteins. Therefore, the expression of the proteins is well balanced between each other and the differing levels of expression of proteins are essential for the method of the present invention and result in a reliable screening, sorting and/or enrichment of cells producing high levels of the protein of interest.

The order of sequences GOI - IRES element - Reporter gene ensures proportional but reduced expression levels of genes behind the IRES element compared to the GOI. The level of reduction depends on the efficiency of the incorporated IRES element. The 2A element ensures equal expression levels of the reporter gene in front of the 2A sequence and the selection marker gene following the 2A sequence. The order of reporter gene and selection marker gene is interchangeable, but the order described here is preferred. This constellation of order of sequences guarantees sufficient expression of the most important gene, the GOI, within the screening and enrichment system. The expression levels of reporter protein and selection marker protein should be reduced on the other hand due to following aspects: first, reduced levels of selection marker protein ensure that only cells expressing at high level produce enough selection marker protein to survive the applied selection pressure. Due to the coupled expression of GOI and selection marker protein this also ensures selection of cells expressing high levels of GOI. Second, reduced expression of reporter protein facilitate the screening and enrichment of only high-producers; this low-level reporter protein expression is especially important for magnetic cell sorting (e.g. magnetic activated cell sorting (MACS^{®g})) technology, in which all cells expressing a certain level of reporter protein on their surface are labelled and enriched. Third, low level expression of reporter protein and selection marker protein reduce the expression burden for the cell and allow the major part of the cell protein producing capacity to be used for the expression of the GOI.

It is an object of the invention to provide a method for screening, and enriching stable eukaryotic cells expressing high levels of a protein(s) of interest, the method comprising
i) transfecting or transducing eukaryotic host cells with at least one polynucleotide comprising a tricistronic expression cassette comprising
   a) a promoter
   b) a gene of interest (GOI),
   c) a reporter gene,
   d) a selection marker gene,
   e) an internal ribosome entry site (IRES) element
   f) a 2A element,
   wherein the order in 5' to 3'direction within said tricistronic expression cassette is: promoter - GOI - IRES element operably linked to the reporter gene or the selection marker gene - 2A element operably linked to the reporter gene or the selection marker gene.
ii) culturing the eukaryotic host cells under conditions so as to express the protein(s) of interest, the reporter protein(s) and the selection marker protein(s) of said at least one polynucleotide in a cell culture selection medium for selecting positively-transfected /transduced cells by means of said selection marker protein(s)
iii) screening, sorting and/or enriching the cells expressing high levels of protein(s) of interest by means of the reporter protein(s).

Methods well known in the art which allow for screening, sorting and/or enrichment of cells by identifying a marker, i.e. the reporter protein, are e.g. methods of cell sorting by flow cytometry (e.g. the fluorescence activated cell sorting (FACS^{™})), the magnetic cell sorting technology (e.g. magnetic activated cell sorting (MACS^{®})) and clone isolation from semi-solid media. Other possible applications for screening and enrichment are known to those skilled in the art. The use of methods of cell sorting by flow cytometry and/or magnetic cell sorting technologies are preferred methods for screening, sorting and/or enrichment of the cells using the method of the present invention. Most referred is the use of the combination of a method of cell sorting by flow cytometry, e.g. FACS^{™}, and a magnetic cell sorting technology, e.g. MACS^{®}, for enrichment of these cells.

### Brief description of the Drawings

FIG 1: Bicistronic expression vector: Expression of GOI under the control of a eukaryotic promoter. GOI and selection maker are expressed in a bicistronic cassette. The vector additionally contains a bacterial amplification site and selection marker.
FIG 2: Tricistronic expression vector: Expression of GOI under the control of a eukaryotic promoter. GOI, reporter gene and selection marker are expressed in a tricistronic cassette. The vector additionally contains a bacterial amplification site and selection marker.
FIG 3: Stable CHO cell lines generated with a bicistronic vector using P5CS selection system in proline-free medium expressing GFP (pAS-16), GFP expression was monitored by flow cytometry. A: percentage of GFP positive cells; B: mean fluorescent intensity
FIG 4: Stable CHO cell lines generated with a bicistronic vector producing a model cytokine, selected by P5CS in proline-free medium and co-expressing GFP. The co-expression of GFP was monitored using flow cytometry: A: percentage of GFP positive cells; B: mean fluorescent intensity
FIG 5: Stable CHO cell lines expressing a model cytokine using P5CS selection generated using either a bicistronic or a tricistronic (co-expression of GFP or membrane-bound GFP) vector cytokine concentrations were monitored by enzyme linked immunosorbent assay (ELISA) over culture time.
FIG 6: CHO-S clones expressing a model cytokine; cells were selected using P5CS and clones were isolated using the co-expressed reporter protein GFP fluorescent in a FACS^{™} sort: Intracellular staining of the model cytokine with cytokine-specific allophycocyanin (APC)-coupled antibody compared to the GFP expression showing a correlation of GOI and reporter protein expression.
FIG 7: Stable CHO cell lines expressing a model cytokine generated using a bicistronic vector selected by different selection markers: P5CS, neomycin resistance, hygromycin resistance and zeocin resistance: Cytokine concentrations were monitored by ELISA over culture time.
FIG 8: Flow cytometry dot blot of a CHO stable cell line day 21 after transfection expressing a model antibody in a two-vector-strategy selected by a combination of of zeocin and P5CS using tricistronic vectors co-expressing GFP and red fluorescent protein (RFP).
FIG 9: CHO clones expressing a model antibody in a two-vector-strategy selected by a combination of zeocin and P5CS using tricistronic vectors co-expressing GFP or RFP isolated from the same starting cell line by either limiting dilution or FACS^{™} sorting. A: Clone distribution after FACS^{™} sorting, B: Clone distribution after limiting dilution, C: Clone analysis of best clones isolated by FACS^{™} sorting, D: Clone analysis of best clones isolated by limiting dilution.
FIG 10: Table comparing the efficiency of the limiting dilution (compare FIG 9B, D) and FACS sorting (compare FIG 9A, C).
FIG 11: Antibody expressing clones isolated by FACS^{™} cloning (compare FIG 9A, C) followed by a second sub-cloning using FACS^{™}; A: Clone analysis of the best isolated clones; B: table showing the efficiency of the first and second FACS^{™} cloning step.
FIG 12: Cytokine expressing clones isolated by FACS^{™} cloning: Clone analysis of the best isolated clones.
FIG 13: Flow cytometry dot blot of a stable antibody expressing CHO cell pool (light chain (LC)-mGFP-zeocin resistance and heavy chain (HC)-RFP-P5CS) before (A) and after (B) MACS^{®} enrichment targeting the co-expressed membrane-bound GFP (mGFP).
FIG 14: MACS^{®} to enrich high producers: stable CHO cell line expressing a model antibody in a two-vector-strategy selected by a combination of zeocin and P5CS a using tricistronic vector co-expressing a membrane-bound GFP and RFP. The stable cell line was enriched four times with MACS^{®} targeting the membrane-bound GFP.
FIG 15: A stable cell line (expressing model antibody in a two-vector-strategy selected by a combination of zeocin and neomycin using tricistronic vectors co-expressing membrane-bound GFP and RFP) was enriched using MACS^{®} technology (compare FIG 14). The enriched cell line was sub-cloned using FACS^{™} sorting; A: Clone analysis of the best isolated clones; B: table showing the efficiency of MACS^{®} enrichment followed by a FACS^{™} cloning step.
FIG 16: MACS^{®} to enrich high producers: stable CHO cell line expressing a model cytokine in a one-vector-strategy selected by P5CS using tricistronic vector co-expressing membrane-bound GFP: The stable cell line was enriched with MACS^{®} tagging the membrane-bound GFP; produced cytokine concentration was monitored by ELISA.
FIG 17: Growth curves of CHO-K1 wild-type cell lines in medium containing proline compared to CHO-K1 cell lines adapted to growth in proline-free medium growing in proline-free medium.
FIG 18: Western Blot analysis of P5CS and glycerinealdehyde 3-phosphate dehydrogenase (GAPDH) expression in different CHO wild-type cell lines (wt) and cell lines adapted to growth in proline-free medium (Pro+). Human embryonic kidney (HEK) cells were used as a positive control.
FIG 19: Different stable CHO cell lines generated using P5CS selection system in proline-free medium. GFP expression of the stable cell lines was monitored by flow cytometry. A: percentage of GFP positive cells; B: mean fluorescent intensity.
FIG 20: Overview of GFP expression of a stable CHO cell line selected with the P5CS selection system and one clone isolated by clone picking from semi-solid medium. Percentage of GFP positive cells and mean fluorescent intensity of the clone are stable with and without selection pressure.
FIG 21: Stable CHO cell lines expressing a model antibody in a two-vector-strategy selected by different selection marker and heavy/light chain combinations using bicistronic vectors.
FIG 22: Stable CHO cell lines expressing a model antibody in a two-vector-strategy selected by a combination of zeocin and P5CS or zeocin and neomycin using bicistronic vectors.
FIG 23: Flow cytometry dot blot of an intracellular staining with a human-kappa-specific fluorescein isothiocyanate (FITC)-coupled and a human-IgG-specific APC-coupled antibody of a stable CHO cell line expressing a model antibody in a two-vector-strategy selected by a combination of (A) of zeocin resistance and P5CS and (B) zeocin resistance and neomycin resistance using bicistronic vectors at day 72 after transfection.
FIG 24: Percentage of RFP/GFP double positive cell of a stable CHO cell lines expressing a model antibody in a two-vector-strategy selected by a combination of zeocin and P5CS using tricistronic vectors co-expressing GFP and RFP.
FIG 25: CHO clones expressing a model antibody in a two-vector-strategy using bicistronic vectors. A: Clone distribution of a limiting dilution selected by a combination of zeocin and P5CS; B: Clone distribution of a limiting dilution selected by a combination of zeocin and neomycin; C: Clone analysis of best clones isolated by limiting dilution selected by a combination of zeocin and P5CS; D: Clone analysis of best clones isolated by limiting dilution selected by a combination of zeocin and neomycin.
FIG 26: Determination of specific productivity of CHO clones expressing a model antibody in a two-vector-strategy selected by a combination of zeocin and P5CS using a bicistronic vector. Clones were isolated by limiting dilution; best 10 clones were adapted to serum-free, proline-free CHO MACS medium and incubated in an orbital shake incubator. Cell numbers and antibody concentration of the culture were determined daily to calculate specific productivities.
FIG 27: Determination of specific productivity of CHO clones expressing a model antibody in a two-vector-strategy selected by a combination of zeocin and P5CS using a bicistronic vector. Clones were isolated by limiting dilution; the best 10 clones were adapted to serum-free, proline-free CHO MACS medium and incubated in an orbital shake incubator. Cell numbers and antibody concentration of the culture were determined daily to calculate specific productivities. Clones were fed daily with CHO MACS CD Feed Supplement.

### Detailed description of the invention

Surprisingly, the polynucleotide comprising a tricistronic expression cassette comprising a gene of interest, a reporter gene, and a selection marker gene, and the presence of an IRES element and a 2A element allows for a fast and easy screening and enriching method. This method for protein expression in eukaryotic cells leads to high production levels of protein(s) of interest.

Therefore, the polynucleotide disclosed herein is well-suited for a method for screening and enriching stable eukaryotic cells expressing high levels of a protein of interest. In a first step suitable eukaryotic host cells are transfected or transduced with polynucleotides, the polynucleotides comprising a tricistronic expression cassette comprising a) a promoter, b) a gene of interest (GOI), c) a reporter gene, d) a selection marker gene, e) an internal ribosome entry site (IRES) element operably linked either to the reporter gene or the selection marker gene, and f) a 2A element operably linked either to the reporter gene or the selection marker gene. In a second step the eukaryotic host cells are cultured under conditions so as to express the protein of interest, the reporter protein and the selection marker protein in a cell culture selection medium suitable for selecting positively-transfected or -transduced cells by means of said selection marker. In a third step the cells expressing high levels of protein of interest are screened, sorted and/or enriched by means of the reporter protein. Methods well known in the art which allow for screening, sorting and/or enrichment of cells by identifying a marker, i.e. the reporter protein, e.g. are the cell sorting by flow cytometry (e.g. FACS^{™}) technology, the magnetic cell sorting technology (e.g. MACS^{®}) and clone picking in semi-solid media. Especially, using sorting and enrichment technologies such as FACS^{™} and MACS^{®} as third step within the method of the present invention result in fast and easy identification of high producers. Unexpectedly, a combination of magnetic- (e.g. MACS^{®}) and flow cytometry- ( e.g. FACS^{™}) based technologies in the third step of the method (enrichment step) results in a particular fast screening and enrichment method.

In addition unexpectedly, it was found that contemplation of a cell line reported to be auxotroph due to a deficiency in both available proline catabolic pathways such as the CHO cell line by the recombinant metabolic enzyme P5CS (1-pyrroline-5-carboxylate synthetase) as selection marker of the polynucleotide comprising the tricistronic expression cassette as disclosed herein results in a combination of both a fast and easy expression system for production of milligram to gram amounts of recombinant proteins, i.e. the protein of interest, and its application to fast screening and enrichment technologies. No permanent use of an inhibitory, toxic drug is used otherwise needed in other protein expression systems.

Further surprisingly, it was found that in the present method the combination of P5CS gene and zeocin resistance gene as selection markers within two different polynucleotides each comprising a tricistronic expression cassette of the as disclosed herein and used to be co-transfected or -transduced into a eukaryotic host cell (two-vector strategy) yields in unexpected high producer rates for expressing hetero- or multimeric proteins such as the heavy and light chain of an antibody or fragments thereof.

### Definitions

A "polynucleotide" as used herein refers to a recombinantly or synthetically produced, polymeric form of desoxyribonucleotides, ribonucleotides or analogs thereof or modified polynucleotides. This term includes double- and single stranded DNA or RNA. The polynucleotide might be integrated into e.g. minicircles, plasmids, cosmids, minichromosomes or artificial chromosomes (e.g. human artificial chromosome (HAC), bacterial artificial chromosome (BAC), yeast artificial chromosome (YAC), PI artificial chromosome (PAC)). The polynucleotide can either be an isolate, or integrated in another nucleic acid molecule e.g. in an expression vector or the chromosome of a eukaryotic host cell. The polynucleotide comprises the tricistronic expression cassette as disclosed herein. Preferentially, the polynucleotide comprising the tricistronic expression cassette is present in a plasmid (non-viral vector) or a viral vector. Vectors are generally derived from plasmids or viruses (viral vectors) that contain necessary genetic signals for replication, as well as additional elements for convenience in inserting foreign DNA or RNA, identifying cells that contain recombinant DNA or RNA, and, where appropriate, expressing the foreign DNA or RNA. If the vector construct is a plasmid, it includes components, which are typically present and necessary for functionality in such plasmids, e.g. it will also include an origin of replication (e.g., the ColE1 origin of replication) and a selectable marker (e.g., ampicillin or tetracycline resistance), for replication and selection, respectively, of the plasmids in bacteria. If the vector is a viral vector such as e.g. retroviral or lentiviral vector, it includes components which are typically present and necessary for functionality in such vectors and which are well known to the skilled person of the art. The vector may contain additional elements such as e.g. enhancer elements (e.g. viral, eukaryotic), introns and viral origins of plasmid replication (e.g. OriP, PyOri, SV40 Ori) for plasmid replication in mammalian cells.

A tricistronic expression cassette as used herein refers to a tricistronic expression unit in the polynucleotide for the simultaneous and coordinated expression of three independent genes in eukaryotic cells. One single promoter allows transcription of all three genes (cistrons). Whereas the first gene is translated in a cap-dependent manner, the subsequent ones utilize intercistronic regions of viral origin, the internal ribosomal entry site (IRES). The second and third genes are encoded as a fusion gene; translation initiation for these proteins is initiated at the IRES element. The cleavage of the two gene products occurs at the 2A peptide sequence during translation. It is an essential part of the present invention that the three genes of the tricistronic expression cassette are a gene of interest, a reporter gene, and a selection marker gene.
The promoter of the tricistronic expression cassette includes signals for DNA or RNA dependent RNA polymerase binding and transcription initiation. The promoters used will be functional in the cell type of the host cell in which expression of the selected sequence is contemplated. A large number of promoters including constitutive, inducible and repressible promoters from a variety of different sources, are well known in the art. With inducible promoters, the activity of the promoter increases or decreases in response to a signal. Among the eukaryotic promoters that have been identified as strong promoters for high-level expression in mammalian cells are the e.g. SV40 early promoter (SV40), adenovirus major late promoter, elongation factor-1 alpha promoter (EF-1α), mouse metallothionein-I promoter, Rous sarcoma virus long terminal repeat, (RSV) and human cytomegalovirus immediate early promoter (CMV). For high-level expression in yeast suitable promoters include e.g. the galactokinase (GAL1) promoter, alcohol oxidase (AOX1) promoter, glutathione-dependent formaldehyde dehydrogenase (FLD) promoter, glyceraldehyde-3-phosphate dehydrogenase (GAP) promoter and TEF1-alpha (TEF1) promoter and for expression in insect cells possible promoter include e.g. the *Autographa californica* nuclear polyhedrosis virus (AcNPV) immediate early (IE-1) promoter, polyhedrin (polh) promoter, metallothionein (MT) promoter, actin 5 (AC5) promoter, p10 and Op1E2 promoter.

A "selectable marker gene" is a gene that allows cells carrying the gene to be specifically selected for or against, in the presence or absence of a corresponding selection agent. E.g., an antibiotic resistance gene can be used as a positive selectable marker gene that allows the host cell transfected/transduced with the gene to be positively selected for in the presence of the corresponding antibiotic; a non-transfected/transduced host cell would not be capable of growth or survival under the selection culture conditions.
Typically, a selectable marker gene will confer resistance to a drug or compensate for a metabolic or catabolic defect in the host cell. A variety of marker genes have been described. For example, selectable genes commonly used with eukaryotic cells conferring resistance to a drug include the genes for aminoglycoside phosphotransferase (APH) encoding resistance to neomycin (G418), the gene for puromycin N-acetyl-transferase (PAC) encoding resistance to puromycin, the gene for hygromycin B phosphotransferase (Hph) encoding resistance to hygromycin B, metallothionein genes encoding resistance to heavy metals and genes encoding resistance to blasticidin S and bleomycins, including phleomycin and zeocin. Selectable genes commonly used with eukaryotic cells encoding for metabolic or catabolic enzymes are e.g. dihydrofolate reductase (DHFR), thymidine kinase (tk), glutamine synthetase (GS), asparagine synthetase and reduced folate carrier (RFC). In addition for selection in yeast genes from the e.g. leucine (e.g. LEU2), tryptophan (e.g. TRP1/2), uracil (e.g. URA3), histidine (e.g. HIS3/4), lysine (e.g. LYS2) or adenine (e.g. ADE2) metabolism are suitable metabolic selection markers. A new example for a selectable marker which compensates for a metabolic deficiency of a eukaryotic host cell is the enzyme P5CS as disclosed herein.

The term "gene of interest" refers to a polynucleotide sequence of any length that encodes a product of interest, i.e. the protein of interest. Typically, the selected sequence will be in the range of from 1-20 kilobases (kb) in length, preferably from 1-5 kb. The gene of interest will be a heterologous gene with respect to the host cell. The selected sequence can be a full length or a truncated gene, a fusion or tagged gene and can be a cDNA, a genomic DNA, or a DNA fragment, preferably, a cDNA. The selected sequence can be the native sequence i.e., naturally occurring form(s), or can be mutated or otherwise modified as desired. These modifications include humanization, codon replacement to optimize codon usage in the selected host cell or tagging. The selected sequence can encode a secreted, cytoplasmic, nuclear, membrane bound or cell surface polypeptide. The "product of interest" includes proteins, polypeptides and fragments and fusions thereof, and peptides, and antisense RNA, miRNA and siRNA. The proteins can be e.g. hormones, cytokines and lymphokines, antibodies, receptors, structural proteins and adhesion molecules, transcription factors and cell signalling proteins, enzymes, viral proteins and fragments thereof. The desired proteins can serve as agonist or antagonist, and/or have therapeutic or diagnostic uses.

The term "reporter gene" as used herein refers to a gene which encodes a gene product suitable for screening, sorting and/or enriching the cells transfected or transduced with the polynucleotide comprising the tricistronic expression cassette of the present invention. The gene product can be any polypeptide or protein suitable for the intended use for screening technologies and can be cytoplasmic, membrane-bound or secreted. Examples of useful enzymes include e.g. beta-lactamase, beta-galactosidase, alkaline phosphatase. Examples of fluorescent agents include green, red, far-red, blue or yellow fluorescent proteins. Examples of luminescent agents include e.g. luciferase proteins (such as firefly, renilla, gaussia luciferase). Examples of cell surface markers include molecules from the cluster of differentiation (CD), artificial epitopes or membrane-bound proteins from agents mentioned above such as fluorescent proteins. The reporter gene product can be truncated or a fusion protein. In a preferred embodiment, the reporter gene is a fluorescent agent, such as green fluorescent protein (GFP) or red fluorescent protein (RFP), and the screening, sorting and/or enriching of the positively-transfected/transduced cells are performed by means of fluorescent cell sorting, for instance cell sorting by flow cytometry such as fluorescence activated cell sorting (FACS^{™}). For FACS^{™} the fluorescent protein such as GFP or RFP may be either cytosolic or membrane-bound. If the screening technology is a magnetic cell sorting technology such as MACS^{®} then the reporter protein has to be membrane-bound (resulting in a cell surface marker) to be suitable for sorting by this technique.
Other common fluorescent protein such as other green and red fluorescent proteins (ZsGreen1, tdTomato, DsRed, AsRed, mStrawberry, mCherry, mOrange), yellow fluorescent proteins (YFP, mBanana, ZsYellow1), cyan fluorescent proteins (CFP, AmCyan1) or blue fluorescent proteins (BFP) or far-red fluorescent proteins (mKate2, HcRed1, mRaspberry, E2-Crimson, mPlum) may be used as well, considering sufficient brightness and distinguishable emission spectra. The fluorescent proteins used may be of enhanced brightness such as eXFP, TagXFP or TurboXFP, wherein X may be any green, red, yellow, blue or far-red fluorescent protein as mentioned above. The term "fluorescent protein" such as GFP as used herein refers also to enhanced forms of that protein such eGFP.

As used herein, "cell culture selection medium" refers to nutrient solution used for growing eukaryotic cells, which contain and express the selection marker gene. Commercially available media such as Ham's F10 (Sigma), Minimal Essential Medium ([MEM], Sigma), RPMI-1640 (Sigma), and Dulbecco's Modified Eagle's Medium ([DMEM], Sigma) are exemplary nutrient solutions. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin^{™} drug, Zeocin^{™} drug), trace elements (defined as inorganic compounds usually present at final concentrations in the micromolar range), vitamins, lipids and glucose or an equivalent energy source. The media are frequently supplemented with serum, e.g., fetal calf or horse serum, as a source of hormones, growth factors and other elements. Any other necessary supplements may also be included at appropriate concentrations that would be known to those skilled in the art. In case of a selection marker gene, which confers resistance to a drug, the cell culture selection medium is supplemented with this defined drug, e.g. selection marker gene, i.e. resistance gene, e.g. zeocin resistance gene and corresponding drug zeocin^{™}. In case of a selection marker gene which compensate for a defect in a host cell, this defined substance is missing in the cell culture selection medium, e.g. proline if PSCS is the selection marker gene and if the cell line used is auxotroph in proline, e.g. a CHO cell line. In case of a co-transfection/transduction with a selection marker gene which confers resistance to a drug on one polynucleotide and a selection marker gene which compensate for a defect in a host cell on another polynucleotide both parameters have to be fulfilled, the presence of the drug and the absence of the respective metabolic substance in the cell culture selection medium.

"Operably linked" refers to a juxtaposition of two or more components, wherein the components so described are in a relationship permitting them to function in their intended manner. For example, a promoter is operably linked to a coding sequence if it acts in cis to control or modulate the transcription of the linked sequence. One example is if an IRES element is operably linked to a gene to be expressed thereby acting in cis to control and modulate the translation of the linked sequence. Another example is if a 2A element is operably linked to a first gene and a second gene is operably liked to the 2A elements facilitating the co-translational "cleavage" of the two genes into separate proteins.

The term "transfection" describes the process of deliberately introducing nucleic acids into cells by non-viral methods. This process may be performed by chemical transfection (e.g. lipofection, cationic polymer transfection, calcium phosphate transfection), electroporation, sono-poration, optical transfection, microinjection or particle-based methods (e.g. magnetofection, gene gun, impalfection).

The term "transduction" describes the process of deliberately introducing nucleic acids into cells by viral methods. This includes viral vector originated from e.g. retroviruses, lentiviruses, adenoviruses, adeno-associated viruses and baculoviruses.

A "producer cell" or "producer cell line" as used herein refers to a cell or cell line, which produces the protein of interest after transfection or transduction using the method of the present invention. A "high level of product" or a "high producer (cell line)" as used herein refers to cells which have higher expression levels of product then most other comparable cells which have been transfected or transduced and treated under equal or similar conditions to produce the protein of interest. The term "pool producer cell line" refers to heterogeneous mixture of cells that originated from more than one single cell whereas the term "clonal producer cell line" or "clone" refers to a cell line that originated from one single cell.

A "host cell" refers to a cell into which a polynucleotide of the invention is introduced. Host cell includes both prokaryotic cells used for propagation of the construct to prepare plasmid (vector) stocks, and eukaryotic cells for expression of the selected sequences such as mammalian cells, insect cells, yeast cells and protozoa. Possible insect host cells include e.g. Spodoptera frugiperda cell lines (Sf9, Sf21), Trichoplusia ni cell lines (Tn5B1-4, High Five) and Drosophila melanogaster cell lines (S2). Yeast cell lines suitable for recombinant protein expression include e.g. Pichia strains (e.g. Pichia pastoris, Pichia methanolica acharomyces) Saccharomyces cerevisiae, Kluyveromyces lactis, Scizosacchromyces pombe, Pichia pastoris and Hansanuela polymorpha. Examples for protozoa host cells include e.g. Tetrahymena thermophila and Leishmania tarentolae. Typically, the eukaryotic cells are mammalian cells such as e.g. chinese hamster ovary (CHO), human embryonic kidney (HEK), baby hamster kidney (BHK), mouse myeloma cell (NS0, Sp2/0), COS, 3T3 or HeLa.

The polynucleotide comprising the tricistronic expression cassette comprises three genes, which are expressed in balance to each other. To achieve a coupled but controlled differing expression of the three proteins the tricistronic expression cassette contains an internal ribosome entry site (IRES) element and a 2A element to initiate translation of coding sequences in a poly-cistronic message. Insertion of an IRES element into polynucleotides of the present invention allows expression of multiple coding regions from a single promoter. IRES elements were first found in the non-translated 5' ends of picornaviruses where they promote cap-independent translation of viral proteins. When located between open reading frames in an RNA, IRES elements allow efficient translation of the downstream open reading frame by promoting entry of the ribosome at the IRES element followed by downstream initiation of translation. A number of different IRES sequences are known including those from encephalomyocarditis virus (EMCV); BiP protein; the Antennapedia gene of Drosophila (exons d and e) as well as those in polio virus (PV). IRES sequences are typically found in the 5' non-coding region of genes.
The term "IRES" as used herein includes any sequence or combination of sequences which work as or improve or decrease the function of a IRES. The IRES(s) may be of viral origin (such as EMCV IRES, PV IRES, or FMDV 2A-like sequences) or cellular origin (such as FGF2 IRES, NRF IRES, Notch 2 IRES or EIF4 IRES) and may be mutated to yield lower expression levels of the following gene. Preferentially the IRES is from EMCV.
The term "2A" (2A element) refers to an about 18-22 amino acid sequence, which can be found in picornaviruses, like FMDV and EMCV, insect viruses and Type C rotaviruses. The highly conserved consensus motif at the C-terminus (D-V/I-E-X-S-N-P-G*P) mediates the cleavage between the C-terminal glycine and the N-terminal proline; this "cleavage" does not require any additional factors like proteases (Szymczak AL et al (2005) Expert Opin. Biol. Ther. 2005; 5: 627-638) and was first thought be an autoproteolytic event. Recently the "cleavage" has been proposed to be a ribosomal-skip mechanism and 2A-peptides are referred to as CHYSELs (*cis*-acting hydrolase elements). The term "2A" as used herein extends to any modification of the sequence of the 2A element, which may improve, increase or have a neutral effect regarding the functionality of the 2A element.

In the polynucleotide comprising the tricistronic expression cassette, the order of the tricistronic sequence may be as follows downstream of the promoter (5'to 3'):
GOI - IRES - reporter gene - 2A element - selection marker gene, or
GOI - IRES - selection marker gene - 2A element - reporter gene.

Although other constellations of the order of the genes and elements are possible, the orders chosen here are superior to other possible orders. These constellations of genes and elements ensure the intended balance of expression levels of the protein of interest, the reporter protein and the selection marker resulting in reliable screening, sorting and/or enriching of cells producing high levels of the protein of interest (high producer cell lines).

Particularly preferred is the order of the tricistronic sequence downstream of the promoter GOI - IRES - reporter gene - 2A element - selection marker gene.
Due to the location of the selection marker at the final position in the tricistronic sequence it is ensured that the complete tricistronic cassette is expressed in order to reach selection marker protein levels that are sufficient to endure the applied selection pressure.

After transfection or transduction of cells with the polynucleotide comprising the tricistronic expression cassette and selection in a cell culture selection medium the cells can be screened, sorted and/or enriched according to all methods known to the person skilled in the art. Preferred are magnetic cell sorting technologies (e.g. MACS^{®}) and flow cytometry sorting technologies (e.g. FACS^{™}). One possible sorting technology is the use of cell sorting by flow-cytometry for the enrichment of pool producer cell lines or the isolation of clonal producer cell lines. For cell sorting by flow-cytometry, e.g. FACS^{™}, antibodies against the reporter protein are used that may be coupled with fluorescence markers that are known to a person of skill in the art, like FITC, phycoerythrin (PE), allophycocyanin (APC), cascade yellow and peridinin chlorophyll protein (PerCP). If the cell transfected or transduced with a fluorescent reporter gene such as GFP, fluorescent activated cell sorting is possible without the need of an antibody staining. Using cell sorting by flow-cytometry (e.g. FACS^{®}) it is possible to analyse and sort at rates up to 1×10⁷ per hours with the option to obtain single cells. Combined with gating techniques it is possible to sort cells according to levels of fluorescence.

Another sorting technology is magnetic cell sorting for the enrichment of pool producer cell lines. The term "magnetic cell sorting" or "magnetic cell sorting process" is used herein to refer to procedures for cell separation (cell sorting) including, but are not limited to, magnetic separation using antibodies linked to colloidal magnetic particles, e.g. superparamagnetic microparticles having a size of 10 to 200 nm (MACS^{®}; Miltenyi et al, 1990, Cytometry 11:231-238) or micron-sized magnetic particles (e.g. 1-10 µm). Methods to separate cells magnetically are commercially available e.g. from Miltenyi Biotec, Bergisch Gladbach, Invitrogen, Stem cell Technologies, in Cellpro, Seattle, or Advanced Magnetics, Boston. Using MACS^{®} it is possible to enrich from up to 2x10¹⁰ cells in one enrichment step.

### Embodiments

The polynucleotide which is used in the present invention, i.e. the method for screening and enriching a recombinant protein expressed in a eukaryotic cell, and in the following embodiments of the invention comprises a tricistronic expression cassette comprising
a) a promoter
b) a gene of interest (GOI),
c) a reporter gene,
d) a selection marker gene,
e) an internal ribosome entry site (IRES) element
f) a 2A element,
wherein the order in 5' to 3'direction within said tricistronic expression cassette is: promoter - GOI - IRES element operably linked to the reporter gene or the selection marker gene - 2A element operably linked to the reporter gene or the selection marker gene.

The orders of genes and elements "promoter - GOI - IRES - reporter gene - 2A element - selection marker gene" and "promoter - GOI - IRES - selection marker gene - 2A element - reporter gene" are preferred to reach a high level expression of the first gene (GOI) and reduced expression levels of the following genes (reporter gene and selection marker) to increase selection pressure and facilitate high-producer selection using magnetic or flow cytometry assisted cell sorting technologies. Most preferred is the order "GOI - IRES - reporter gene - 2A element - selection marker gene" in the tricistronic expression cassette downstream of the promoter. The construction of a suitable expression vector containing the described tricistronic expression cassette is described in Example 1.

The polynucleotide described above and used in the present invention can be a polynucleotide, wherein the selection marker gene encodes for a metabolic enzyme or a resistance protein for an antibiotic drug. Some selection marker genes used in the present invention of the tricistronic expression cassette for screening, sorting and enriching high producer cells are the genes conferring resistance to hygromycin B, puromycin, neomycin (G418) and zeocin as well as the gene for the catabolic enzyme P5CS. Use of P5CS as selection marker is possible if a cell line is used reported to be proline auxotroph due to a deficiency in both available proline catabolic pathways such as the CHO cell line.

The polynucleotide described above and used in the present invention can be a polynucleotide, wherein the reporter gene encodes for a cytosolic or membrane-bound reporter protein.
The reporter protein can be any polypeptide or protein suitable for the intended use for screening technologies. It is preferred that the reporter protein is a fluorescent protein, such as the green fluorescent protein (GFP) or red fluorescent protein (RFP). In a two-vector strategy a combination of an enhanced GFP and enhanced RFP is preferred. The screening, sorting and/or enriching of the positively-transfected/transduced cells are performed by means of fluorescent cell sorting, for instance by flow cytometry analysis and sorting such as FACS^{™}. For cell sorting by flow-cytometry (FACS^{™}) the fluorescent protein such as GFP or RFP may be either cytosolic or membrane-bound. If the screening technology is a magnetic cell sorting technology such as MACS^{®} then the reporter protein has to be membrane-bound (resulting in a cell surface marker, which may be stained with an magnetic bead-coupled antibody) to be suitable for sorting by this technique.
Cell surface markers such as cluster of differentiation (CD) markers (for example CD4), LNGFR or artificial epitopes are suitable as well but require a staining with antibodies that are coupled directly or indirectly (e.g. via biotin - streptavidin system) with fluorescence markers (FITC, phycoerythrin (PE), allophycocyanin (APC), cascade yellow and peridinin chlorophyll protein (PerCP)) for each flow cytometry analysis or flow cytometry-based cell sort or magnetic-based cell sorting.

All polynucleotides comprising the tricistronic expression cassette as described above are intended to use in methods for screening, sorting and/or enriching high producer cells lines and may be used in a one-vector or multi-vector strategy.

Therefore, in one embodiment of the present invention, the polynucleotide(s) described above are used within a method for screening and enriching stable eukaryotic cells expressing high levels of a protein(s) of interest, the method comprising
a) transfecting or transducing suitable eukaryotic host cells with the polynucleotide(s) of the present invention
b) culturing the eukaryotic host cells under conditions so as to express the protein(s) of interest, the reporter protein(s) and the selection marker protein(s) in a cell culture selection medium suitable for selecting positively-transfected/transduced cells by means of said selection marker(s)
c) screening, sorting and/or enriching the cells expressing high levels of protein(s) of interest by means of the reporter protein(s).

Methods well known in the art which allow for screening, sorting and/or enrichment of cells by identifying a marker, i.e. the reporter protein, are e.g. the cell sorting by flow cytometry (FACS^{™}) technology, the magnetic-based cell sorting (e.g. MACS^{®}) and clone picking in semi-solid media. Using sorting and enrichment technologies such as flow cytometry- (e.g. FACS^{®}) and/or magnetic-based (e.g. MACS^{®}) as third step (step c) within the method of the present invention result in fast and easy identification of high producers (see examples 3 and 4). Especially preferred is the combination of a magnetic cell sorting enrichment, e.g. MACS^{®} enrichment, of the selected stable producer cell line to generate a high producing pool producer cell lines followed by flow cytometry sorting, e.g. FACS^{™}, to isolate high producing clonal producer cell lines (see example 5).

In another embodiment of the present invention, the polynucleotide used in a one-vector strategy in the method of the invention is a polynucleotide as described above, wherein the selection marker gene is P5CS gene, the reporter gene is a fluorescent gene such as GFP gene, enhanced GFP gene or a membrane-bound GFP gene. Said method for screening and enriching stable eukaryotic cells expressing high levels of a protein of interest comprises
a) transfecting or transducing suitable eukaryotic host cells which are proline auxotroph with the said polynucleotide
b) culturing the eukaryotic host cells in proline-free cell culture selection medium under conditions so as to express the protein of interest, the reporter protein and the selection marker protein
c) screening, sorting and/or enriching the cells expressing high levels of protein of interest by means of the reporter protein.

The most preferred constellation of the genes and elements within the tricistronic expression cassette for the expression of one recombinant protein (one-vector strategy) in a host cell is:
promoter - gene encoding for a recombinant protein or fragment thereof (GOI)- gene encoding for a membrane-bound fluorescent gene such as GFP gene - P5CS gene, wherein
the IRES element is operably linked to the fluorescent gene, and wherein the 2A element is operably linked to the P5CS gene (see Example 2).

Preferentially, in a one-vector strategy the gene encoding a recombinant protein (GOI) is a monomeric protein such as a monomeric cytokine. But of course the GOI may be any gene or fragment thereof coding for a protein or fragment thereof which one wants to screen, sort or enrich for.

Use of P5CS as selection marker is possible if a cell line is used reported to be proline auxotroph due to a deficiency in both available proline catabolic pathways such as the CHO cell line. Tests in proline-free media and Western Blot analyses revealed that the CHO-S cell line, a suspension-adapted clone of the parental CHO-K1, is suited best for the proline selection system. Using CHO-S selection incubation times and expression levels are comparable to antibiotic selection with neomycin or hygromycin but without the disadvantages of the cytotoxicity of said antibiotic drugs. Moreover, a producer rate of over 90% is reached in less than 15 days expressing the green fluorescent protein (GFP) and isolated clones are stable with and without selection pressure for several months (see Example 5).

In a further embodiment of the present invention, the polynucleotides as described above are used in a multi-vector strategy (i.e. a two-vector or more-vector-strategy), i.e. the polynucleotides used in the method of the invention are polynucleotides as described above, wherein in the polynucleotides are different in GOIs, reporter genes and selection marker genes. Said method for screening and enriching stable eukaryotic cells expressing high levels of a protein of interest comprises
a) transfecting or transducing suitable eukaryotic host cells with said polynucleotides
b) culturing the eukaryotic host cells under conditions so as to express the proteins of interest, the reporter proteins and the selection marker proteins in a cell culture selection medium suitable for selecting positively-transfected/transduced cells by means of said selection markers
c) screening, sorting and/or enriching the cells expressing high levels of proteins of interest by means of the reporter proteins.

In a further embodiment of the present invention, the polynucleotides as described above are used in a two-vector strategy, i.e. the polynucleotides used in the method of the invention are polynucleotides as described above, wherein in a first polynucleotide of the present invention the selection marker gene is P5CS gene and wherein in a second polynucleotide of the present invention the selection marker gene is the zeocin resistance gene. Said method for screening and enriching stable eukaryotic cells expressing high levels of a protein of interest comprises
a) transfecting or transducing suitable eukaryotic host cells which are proline auxotroph with the first and the second polynucleotide
d) culturing the eukaryotic host cells in proline-free and zeocin-containing cell culture selection medium under conditions so as to express the proteins of interest, the reporter protein and the selection marker protein
e) screening, sorting and/or enriching the cells expressing high levels of protein of interest by means of the reporter proteins.

A combination of P5CS and zeocin selection enables us to express recombinant antibodies in a two-vector-strategy. Again, a producer rate of more than 90% is obtained within 18 days (see Example 6).
Preferentially, in such a two-vector-strategy two different GOIs are used for the two different polynucleotides wherein the two GOIs encode for different subunits of the same di- or-multimeric protein. But of course the GOIs may be any gene or fragment thereof coding for a protein or fragment thereof which one wants to screen, sort or enrich for.

In a further embodiment of the present invention, the polynucleotides used in the method of the invention are polynucleotides as described above, wherein in a first polynucleotide the selection marker gene is P5CS gene, the reporter gene is a fluorescent gene such as RFP gene or a membrane-bound GFP gene, and GOI is a gene encoding for a heavy chain of an antibody, and wherein in a second polynucleotide the selection marker gene is the zeocin resistance gene, the reporter gene is a different fluorescent gene as used in the first polynucleotide and GOI is a gene encoding for a light chain of an antibody. The fluorescent genes can encode cytoplasmic, membrane-bound or secreted proteins. If magnetic cell sorting is used to screen, sort and/or enrich the cells, one or both fluorescent genes are genes encoding membrane bound fluorescent proteins. As described in EP819250B1 using MultiSort MicroBeads or other bead types suitable for a release mechanism it is possible to use multiple rounds of magnetic enrichment, first enriching via one surface molecule, followed by releasing bound magnetic particles, followed by a second enrichment step targeting a second, different surface molecule. If the reporter protein is a secreted protein technologies such as e.g. MACS^{®} Cytokine Secretion Assay (EP0667896B1), clone picking from semi-solid media or enrichment by flow cytometry using a Surface Affinity Matrix (Borth N et al (2000) Biotech Bioeng; 71: 266-273) may be used to enrich target cells.
Said method for screening and enriching stable eukaryotic cells expressing high levels of a protein of interest comprises
a) transfecting or transducing suitable eukaryotic host cells which are proline auxotroph with the first and the second polynucleotide
b) culturing the eukaryotic host cells in proline-free and zeocin-containing cell culture selection medium under conditions so as to express the proteins of interest, the reporter protein and the selection marker protein
c) screening, sorting and/or enriching the cells expressing high levels of protein of interest by means of the reporter proteins.

The preferred constellations of the genes within the two expression cassettes are for the expression of two recombinant proteins, e.g. antibody heavy and light chain:
First polynucleotide: promoter - gene encoding for a heavy chain of Ab - RFP gene or GFP gene - P5CS gene, wherein the IRES element is operably linked to the gene encoding for the fluorescent protein, and wherein the 2A element is operably linked to the P5CS gene.
Second polynucleotide: promoter - gene encoding for a light chain of Ab - GFP gene or RFP gene - zeocin resistance gene, wherein the IRES element is operably linked to the gene encoding for a fluorescent protein, and wherein the 2A element is operably linked to zeocin resistance gene, and, wherein in said second polynucleotide the reporter gene is GFP gene, if in said first polynucleotide the reporter gene is RFP gene, or the reporter gene is RFP gene, if in said first polynucleotide the reporter gene is GFP gene. Both fluorescent proteins might be cytosolic or membrane-bound, or one fluorescent gene might be cytosolic and the other might be membrane-bound, depending on the application as specified above.

The terms "heavy chain of an antibody" and "light chain of an antibody" comprise both intact molecules, i.e. complete single chain antibodies, or a fragment thereof or any variant of an antibody known in the art.

In another embodiment of the present invention the first and second polynucleotides of the proceeding embodiment are used in the method of the proceeding embodiment but the GOI of the first polynucleotide and the GOI of the second polynucleotide are replaced by other genes encoding proteins other than antibodies. Preferentially, these proteins are hetero-dimeric or hetero-multimeric proteins having two or more subunits, e.g. receptors such as G protein (βγ subunit dimer) and estrogen receptor (αβ subunit dimer), cytoskeleton proteins such as microtubules (αβ-tubulin dimer), adhesion molecules such as integrins (αβ subunit dimer) or cytokines such as the interleukin-12 family which consists of IL-23 (p35 and p40 dimer), IL-23 (p40 and p19 dimer), IL-27 (EBI3 and p28 dimer) and IL-35 (p35 and EBI3 dimer).

### Examples

Hereinafter, the present invention is described in more detail and specifically with reference to the examples, which however are not intended to limit the present invention.

### Example 1: Vector construction of pMACS-CHO vector

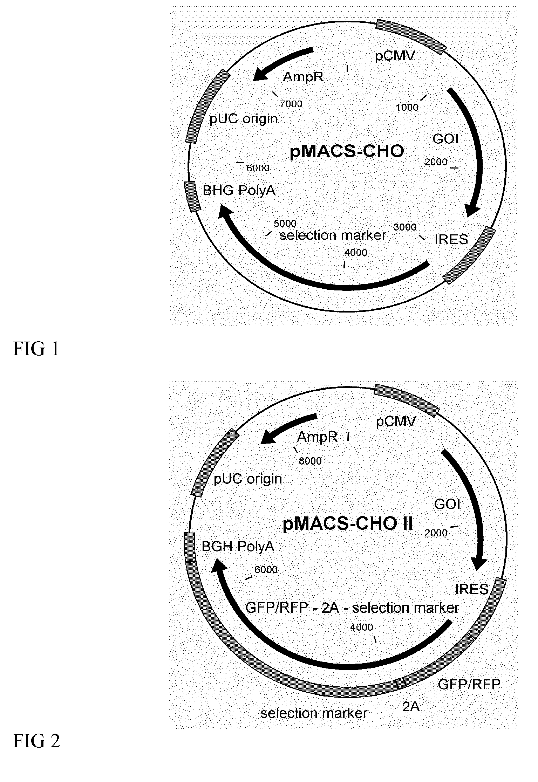

The pMACS 4-IRES.II (Miltenyi Biotec, #130-091-888) vector was used as a backbone for the pMACS-CHO vector. Digestion with restriction enzymes (New England BioLabs), agarose gel electrophoresis followed by gel extraction (High Pure PCR Product Purification Kit, Roche) and ligation (Rapid DNA Dephos & Ligation Kit, Roche) according to manufacturers instructions was used to remove the multiple cloning site, CD4 cDNA and PolyA. Synthetically assembled (GeneArt) new multiple cloning sites were integrated before and after the IRES element. A bovine growth hormone (BGH) PolyA sequence was integrated behind the second multiple cloning site to generate the bicistronic pMACS-CHO vector (FIG 1). For the generation of a tricistronic pMACS-CHO II vector the DNA sequence coding for the 2A peptide from the Thosea asigna virus (Szymczak AL and Vignali DA, Expert Opin Biol Ther. 2005; 5(5): 627-638) was integrated in the second multiple cloning site (FIG 2). Genes of interest (GOIs), that were integrated into the first multiple cloning site behind the promoter and in front of the IRES element, are the cDNA sequence of a fluorescent protein, the cDNA of a human cytokine and the cDNA of a humanized antibody with an IgG1 heavy chain (HC) sequence and a kappa light chain (LC) sequence. These GOIs were chosen for the expression as model recombinant proteins, but other recombinant protein sequences or fragments thereof would be suitable as well. All sequences were codon optimized for expression in human cells (DNA2.0). All GOI sequences compromise the Kozak sequence "GCC GCC ACC" before the start codon.

Selection markers integrated at the second multiple cloning site behind the IRES element in the bicistronic vector were synthetically synthesized (DNA2.0) as cDNA sequences, codon optimized for expression in human cells and contain the Kozak sequence described above as well as start and stop codons. If integrated at the third position of the tricistronic vector behind the 2A element the same sequence was used but Kozak sequence and start codon were removed and the selection marker genes were cloned in frame with the sequence in the second multiple cloning site and the 2A sequence.

Used selection markers are the mouse P5CS (Aldh18a1) transcription variant 1, cDNA sequence (NCBI Reference Sequence NM_019698.2, nucleotide 249-2636), the neomycin resistance gene (neomycin phosphotransferase gene) (NCBI GeneBank: U55761, nucleotide 2629-3423), the zeocin resistance gene (NCBI GenBank: FJ716125.1, nucleotide 2000-2374) and the hygromycin resistance gene (NCBI GenBank: JQ513372.1, nucleotide 1367-2404). Reporter proteins integrated at the second multiple cloning site in the tricistronic vector between IRES and 2A element contain a Kozak sequence as described above and a start codon but do not contain a stop codon and are integrated in frame with the following 2A sequence and selection marker sequence to generate one open reading frame. The used 2A sequence contains a serin-glycin linker and the 2A peptide sequence from the Thosea asigna virus: TCTGGCTCCGGAGAGGGCCGGGGCTCTCTGCTGACCTGTGGCGACGTGGA GGAGAACCCCGGCCCC. Used reporter protein sequences are the cDNA sequence of the enhanced green fluorescent protein (NCBI GeneBank: U55761, nucleotide 97-816) and the red fluorescent protein TagRFP (Evrogen, pTagRFP-N, FP142, nucleotide 679-1389). To generate a membrane-bound GFP the sequence of the enhanced green fluorescent protein (NCBI GeneBank: U55761, nucleotide 97-816) was integrated in frame into the multiple cloning site of the pDisplay^{™} vector (Invitrogen, V660-20) and the open reading frame consisting of Ig kappa leader, HA tag, newly integrated GFP, mycC tag and the PDGFR transmembrane domain without a stop codon were integrated into the tricistonic pMACS-CHO II vector.

### Example 2: Recombinant protein expression in CHO cells using the enzyme P5CS as a selection marker in proline-free medium

The cDNA of the green fluorescent protein (GFP) was cloned into the bicistronic pMACS-CHO containing the P5CS gene as a selection marker as described in Example 1. The human cytokine gene was cloned into the bicistronic pMACS-CHO or tricistronic pMACS-CHO II vector containing different selection marker genes and the GFP or the membrane-bound GFP as reporter protein genes as described in Example 1. CHO-S cells (FreeStyle^{™} CHO-S Cells, R800-07, Invitrogen) were transfected using Fugene HD transfection reagent (Roche) according to the manufacturer's instructions. 24 hours after transfection cells were expanded and selection pressure, medium without proline (MEM (Simga, M2279) supplemented with 4mM L-glutamine (PAA M11-004), 5% dialyzed FBS (PAA A15-507) and ITS+3 (Sigma I2771)) for P5CS selection or medium containing appropriate concentrations of antibiotics for antibiotic selection (MEM (Simga, M2279) supplemented with 4mM L-glutamine (PAA M11-004), 40mg/L proline (Sigma, P5607), 5% dialyzed FBS (PAA, A15-507), ITS+3 (Sigma, I2771) and 100/µg/mL zeocin^{™} (Invivogen, ant-zn-5), 800µg/mL G418 (Merck, 345812) or 800µg/mL hygromycin B (PAA, P02-015), was applied. Selection media were changed once per week until stable cell lines grew up. Stable production cell lines were routinely passaged every 3-4 days in selection medium.

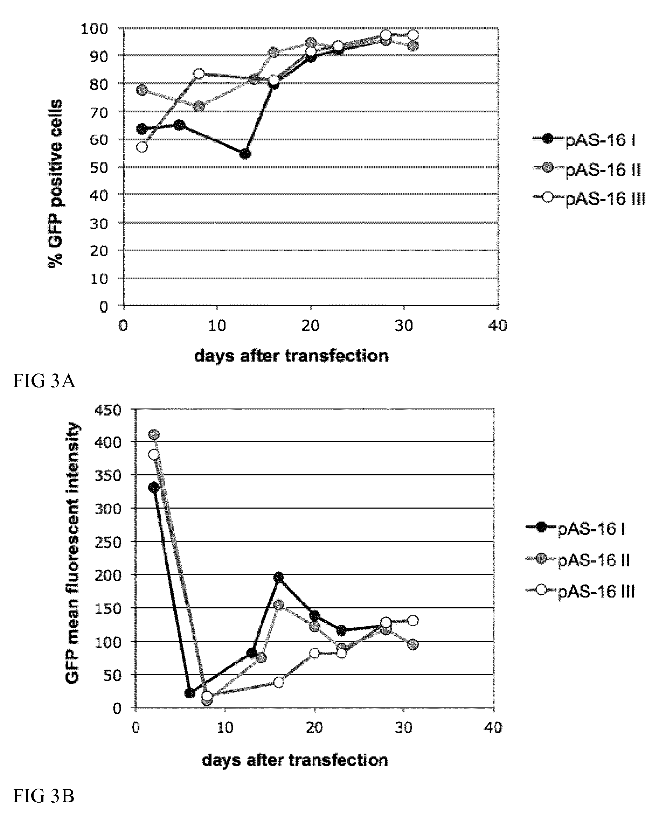

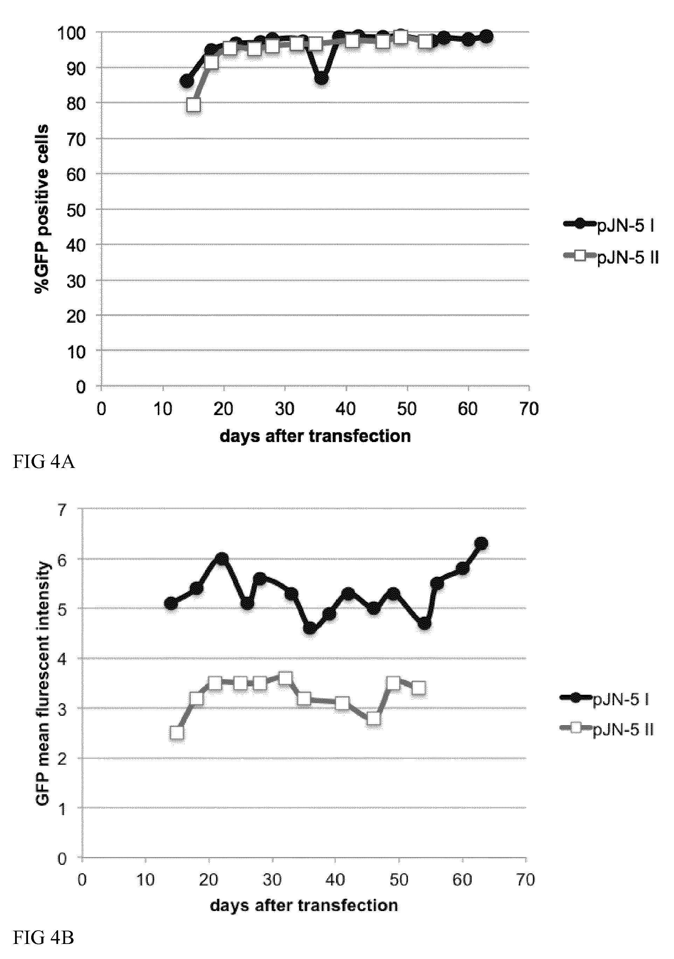

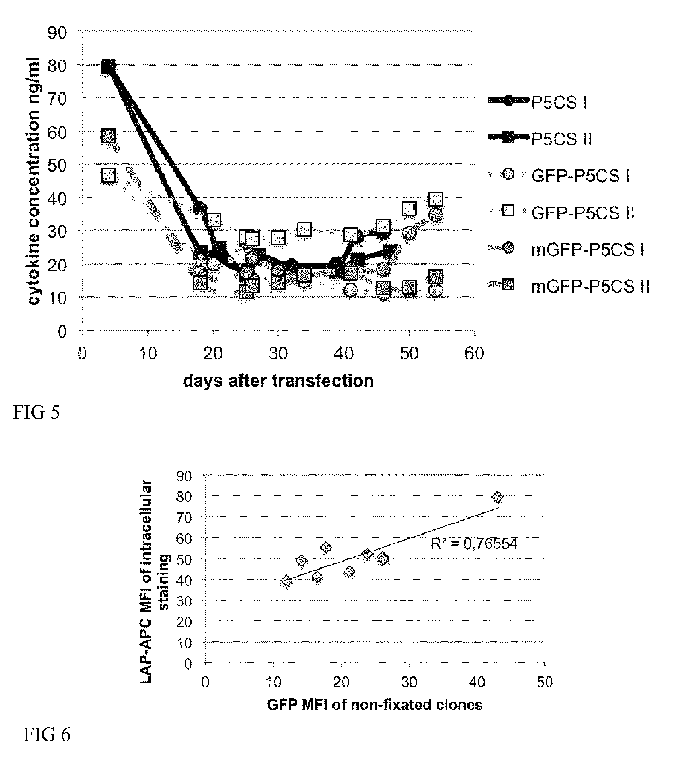

Already 15 to 20 days after transfection stable producer cell lines expressing GFP selected by P5CS selection (pAS-16) monitored using MACSQuant^{®} Analyzer (Miltenyi Biotec) reach producer rates of over 90% with reproducible mean fluorescent intensities (FIG 3). Similar producer rates can be seen in stable producer cell lines selected by P5CS selection expressing a human cytokine and co-expressing GFP (pJN-5). As early as 18 days after transfection producer rates of over 90% are reached, analysed by monitoring GFP co-expression using the MACSQuant^{®} Analyzer (Miltenyi Biotec) (FIG 4). Mean fluorescent intensities of the GFP expressed as a reporter protein (pAS-16) compared to the mean fluorescent intensity as GOI (pJN-5) are reduced 25-fold due to the vector design (FIG 3B and FIG 4 B). The fluorescent intensity of the reporter protein GFP is still high enough for MACS^{®} enrichment or FACS^{™} sorting, but reduced to avoid additional production burden for the cells. Therefore the additional integration of a GFP (GFP-P5CS) or membrane-bound GFP (mGFP-P5CS) in the tricistronic pMACS-CHO II vector has no negative effect on the expression of a cytokine using P5CS selection (FIG 5) compared to the bicistronic vector without an additional fluorescent protein (P5CS), but enables the application of different sorting and enrichment strategies for selection of high-producers.

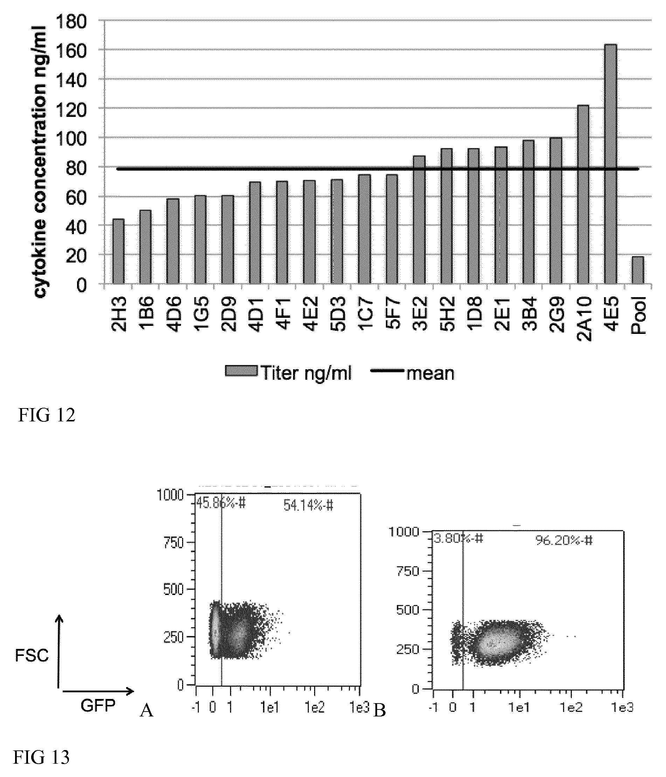

To demonstrate the correlation of expression levels of the GOI and the fluorescent protein, intracellular levels of the cytokine and the fluorescent protein were analysed. CHO-S clones expressing the model cytokine, selected by P5CS in proline-free media and clone isolation by FACS^{™} sorting according to GFP fluorescence (see Example 3 and FIG 12) were fixed and permeabilize (Inside Stain Kit, Miltenyi Biotec, #130-090-477) and stained with anti-LAP-APC (Miltenyi Biotec, # 130-096-573) according to the manufacturer's instructions. APC fluorescence of the fixated and stained cells and GFP fluorescence of non-fixated cells were analysed using the MACSQuant^{®} Analyzer (Miltenyi Biotec). The intracellular cytokine levels, measured as APC fluorescence in the intracellular staining, and GFP fluorescence of the reporter protein show a strong correlation (FIG 6). The results demonstrate the proportional expression of GOI and reporter protein, which can be used to identify and enrich high-producer in a rapid and simple manner.

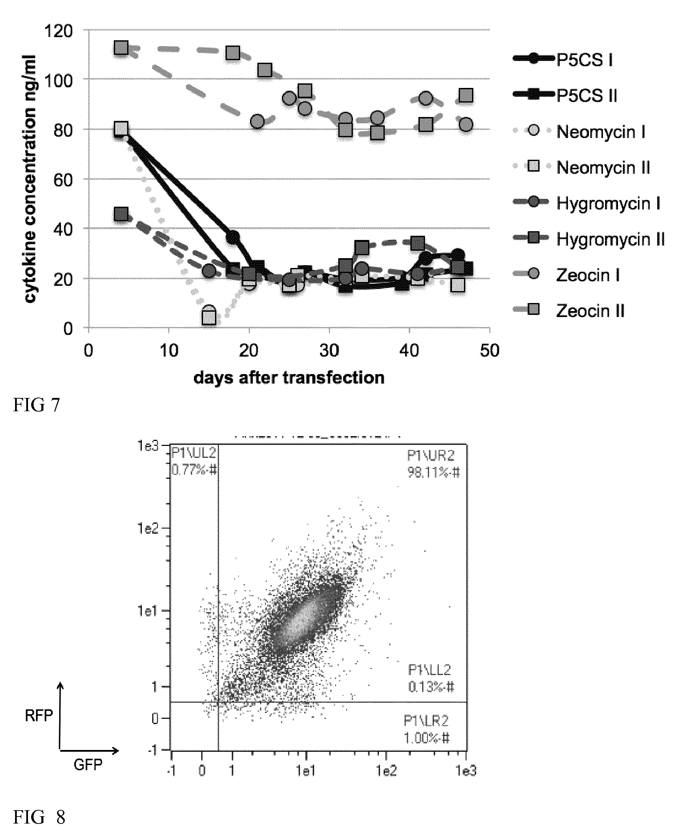

To compare different selection systems the expression of the model cytokine of stable producer cell lines selected by different selection markers was monitored. Cytokine secretion was analysed by seeding the stable cell lines at every passage in an additional flask and letting these cells grow over maximum cell density. Human cytokine titers of the supernatant were determined by ELSIA (Human/Mouse TGF beta1 ELISA Ready-SET-Go!, eBioscience, 88-7344) according to the manufacturer's instructions. Produced concentration of the cytokine in CHO-S cells using neomycin resistance, hygromycin resistance or P5CS selection in a bicistronic pMACS-CHO vector is comparable. Selection using the zeocin resistance leads to cytokine concentration about twice as high (FIG 7).

### Example 3: Clone selection using FACS^{™} sorting

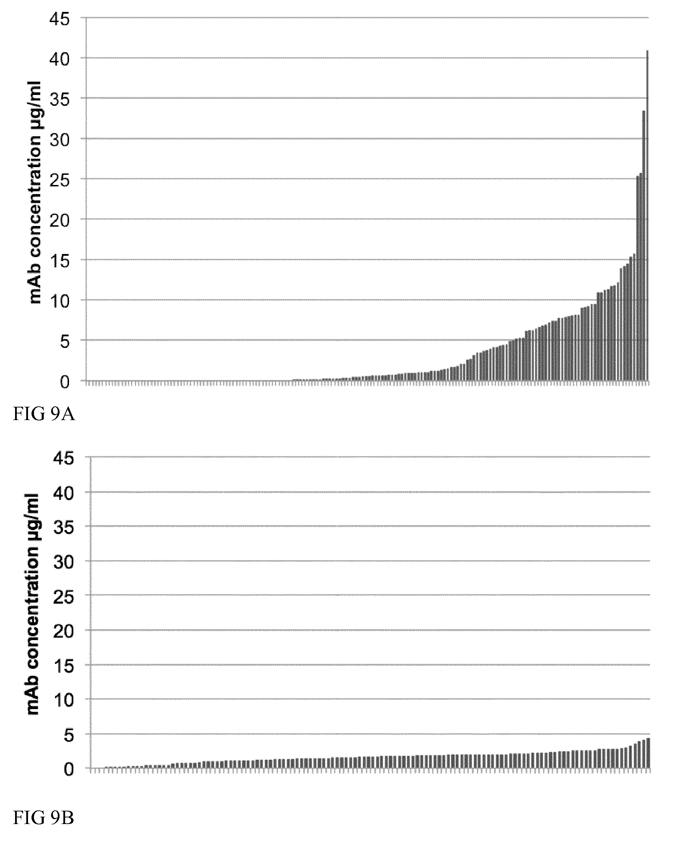

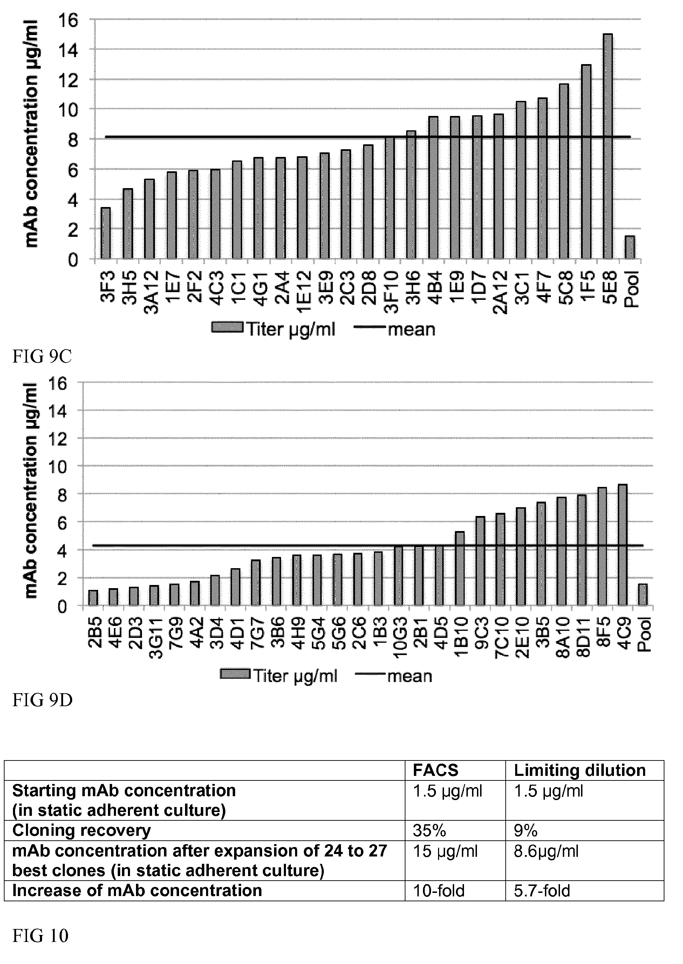

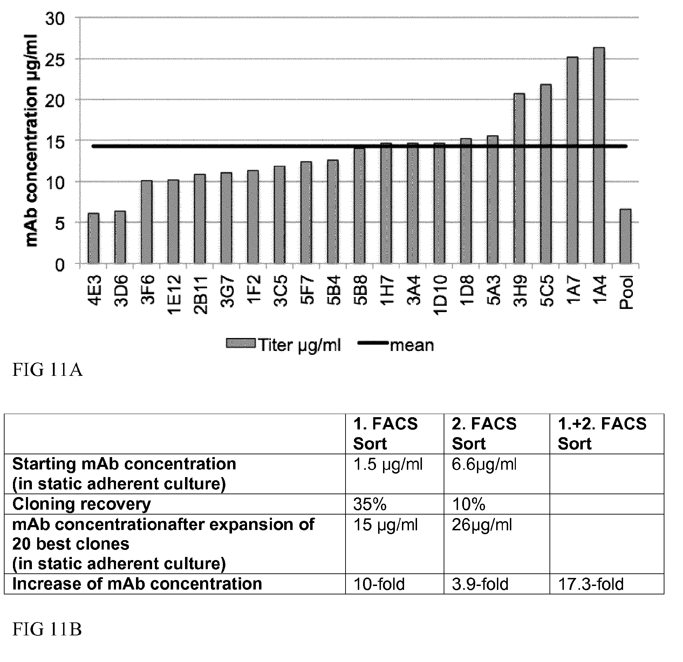

The cDNAs of heavy chain (HC) and light chain (LC) of a model antibody were cloned into two different tricistronic pMACS-CHO II vectors containing the P5CS gene or zeocin resistance gene as a selection marker co-expressing RFP or GFP (LC-RFP-zeocin resistance and HC-GFP-P5CS) as described in Example 1. CHO-S (FreeStyle^{™} CHO-S Cells, R800-07, Invitrogen) cells were co-transfected using Fugene HD transfection reagent (Roche) according to the manufacturer's instructions. 24 hours after transfection cells were expanded and selection pressure, medium without proline containing appropriate concentrations of zeocin^{™} (MEM (Simga, M2279) supplemented with 4mM L-glutamine (PAA, M11-004), 5% dialyzed FBS (PAA, A15-507), ITS+3 (Sigma, I2771) and 100/µg/mL zeocin^{™} (Invivogen, ant-zn-5)) was applied. The generated stable cell line had a producer rate of 98% (FIG 8), monitored by double-expression of RFP and GFP using MACSQuant^{®} Analyzer (Miltenyi Biotec) 21 days after transfection. Productivity was measured as antibody concentration in cell culture supernatant of cells with maximum cell densities. At day 21 the generated cell line produced of antibody concentrations of 1,4µg/ml as analysed by ELISA (coating antibody: IgG (Fc) (anti-human, clone JDC-10) Beckman Coulter 733164; detection antibody: anti-human kappa-HRP Miltenyi Biotec in-house). The stable cell line was FACS^{™} sorted (BD Bioscience Influx^{™} Cell Sorter): The 0.8% brightest RFP and GFP double positive cells were sorted as single cells into 96-well plates. In parallel a limiting dilution of the same stable cell line was performed; 0.5 cell per 96-well was seeded. After 11 days 96-well plates were screened for formation of visible colonies. The antibody concentration of the supernatant of these wells was determined in an ELISA (FIG 9A, B). The best 24 to 29 clones of the screened 140-170 clones were expanded into T75 flasks and analysed for growth and productivity (FIG 9C, D). The highest producing clone from the limiting dilution produced an antibody concentration of 8.6µg/mL, which equates to a 5.7-fold increase (FIG 9 D). In comparison the highest producing clone from the FACS^{™} sorting produced an antibody concentration of 15µg/ml, which equates to a 10-fold increase (FIG 9C). The produced antibody concentration of all 20 expanded clones was 4.3µg/ml for the limiting dilution clones and 8.1µg/ml for the FACS^{™} sorted clones. In summary FACS^{™} sorted clones have a 1.7-fold (highest producing clone) to 1.9-fold (mean clone distribution) higher productivity compared to clones isolated by limiting dilution (FIG 10).
One clone (5C8) isolated by the first FACS^{™} sort was subjected to a sub-cloning step. As described above this clone was FACS^{™} sorted, the 96-well plates screened and the best clones were expanded. The highest producing clone of this second FACS^{™} sort produced an antibody concentration of 26µg/ml (FIG 11), which equates to a 3.9-fold increase in the second cloning step; the total titer increase from both cloning step is 17.3-fold (FIG 11). The mean antibody concentration produced of all 20 expanded clones of the second FACS^{™} sorting step was 14µg/ml.

To verify the universal applicability of the tricistronic vector for clone enrichment using FACS sorting, the clone isolation process was in addition performed with a stable CHO-S cell line expressing a human cytokine as a second model protein and GFP as a reporter protein selected by the P5CS system (FIG 5, GFP-P5CS I). The cell line generation is described in Example 2. As described above for the antibody-producing cell line the FACS sort, the 96-well plate screen and the expansion of the best clones was repeated for this cytokine-producing cell line. The highest producing clone of this FACS^{™} sort produced a cytokine concentration of 163 ng/ml, which equates to a 8.8-fold increase in this second cloning step; the mean cytokine concentration produced of all expanded clones was 79 ng/ml (compare FIG 12). These results of clone isolations from cell lines expressing different model proteins in a one or two vector strategy demonstrate the advantage of the tricistronic vector system for FACS^{™} -based isolation of high-producing clones.

### Example 4: High-producer enrichment by MACS^{®}

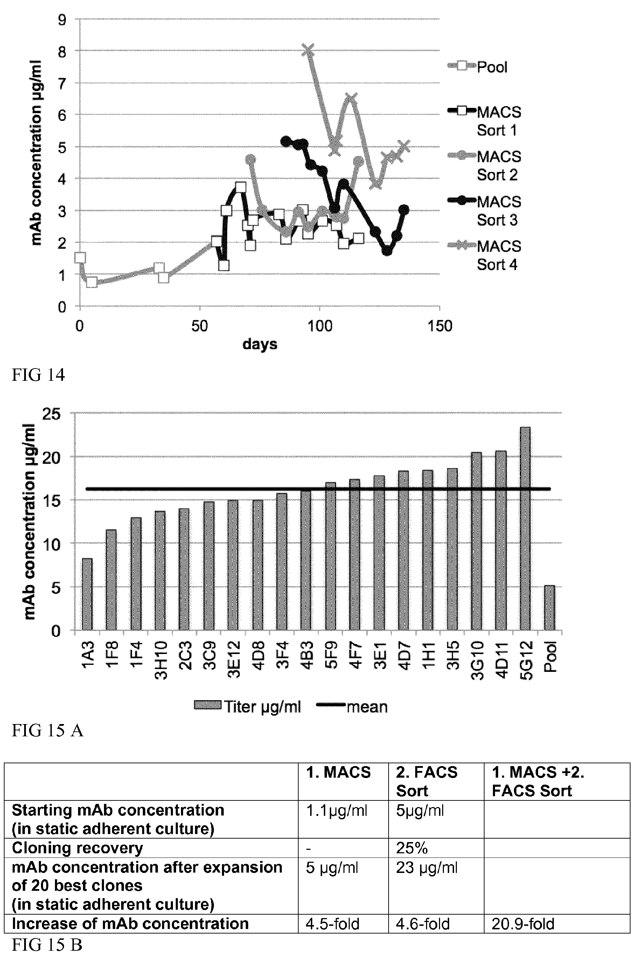

The cDNAs of heavy chain (HC) and light chain (LC) of a model antibody were cloned into two different tricistronic pMACS-CHO II vectors containing the P5CS gene or zeocin resistance gene as a selection marker co-expressing RFP or membrane-bound GFP (LC-mGFP-zeocin resistance and HC-RFP-P5CS) as described in Example 1. Stable cell lines were generated as described in Example 3. The generated stable cell line produced antibody concentrations of around 1µg/ml. The cell line was enriched for high-producers using MACS^{®} technology. Single cell suspension was labelled with a polyclonal rabbit-anti-GFP antibody (Clontech, 632460, Living Colors® Full-Length A.v. Polyclonal Antibody) and anti-rabbit IgG MicroBeads (Miltenyi Biotec, 130-048-602) according to the manufacturer's instructions. To avoid false-positive staining due to Fc receptor interactions, the FcR Blocking Reagent, mouse (Miltenyi Biotec 130-092575) was applied prior to antibody labelling. Cells were loaded on an MS column (Miltenyi Biotec, #130-042-201) placed in the magnetic field of an OctoMACS™ Separator (Miltenyi Biotec). The column was washed to remove GFP-negative and low expressing cells. Magnetically labelled high-positive GFP cells were retained within the column and eluted as positively selected cell fraction after removing the column from the magnet. To increase stringency the eluted cell can be loaded on a second column, washing and elution is identical to the first column. Separation efficiency is monitored by GFP expression in the starting, wash and elution fraction using MACSQuant^{®} Analyzer (Miltenyi Biotec). Eluted cells are cultured in the described selection medium. As an example in the first single enrichment step of the described stable cell line the percentage of GFP expressing cells could be increased from 52% (FIG 13 A) to 96% (FIG 13 B). Due to the low expression of GFP in the tricistronic construct only cells expressing high levels of GFP are retained which is demonstrated by an increase of mean fluorescent intensity from 2.5 to 5.2 (FIG 13A and B). Due to the coupled expression of GOI and mGFP it was possible to increase the produced antibody concentration about 2-fold (FIG 14 Pool to MACS Sort 1). The enriched cell line was expanded and repeatedly enriched. For MACS Sort 3 and 4 the more stringent enrichment using to sequential MS columns described above was used which resulted in a higher GFP mean fluorescent intensity and produced antibody concentration. The final enriched production cell line (FIG 14, MACS Sort 4) produced an antibody concentration of 8µg/ml, which was reduced to 5µg/ml during the following 40 days of culture (FIG 14). This results in a 4.5-fold enrichment.
A MACS^{®} pre-enriched producer cell line is well suited for FACS^{™} clone selection. The described MACS Sort 4 (FIG 14) producer cell line was FACS sorted and the clones analysed as described in Example 3. The 20 best clones were expanded for further analysis. The mean produced antibody concentration of these clones was 16µg/ml, the maximum produced antibody concentration 23µg/ml, which equates to a 4.6-fold increase in the FACS^{™} cloning step (FIG 15). The total increase of antibody production from MACS^{®} enrichment and FACS^{™} sort is 20.9, which is similar to the results obtained from the two repeated FACS^{™} sorting steps described in Example 3. The advantage of the combination of MACS^{®} enrichment and FACS^{™} sort is the reduction of one time-consuming clone screening process compared to repeated FACS^{™} sorting steps.

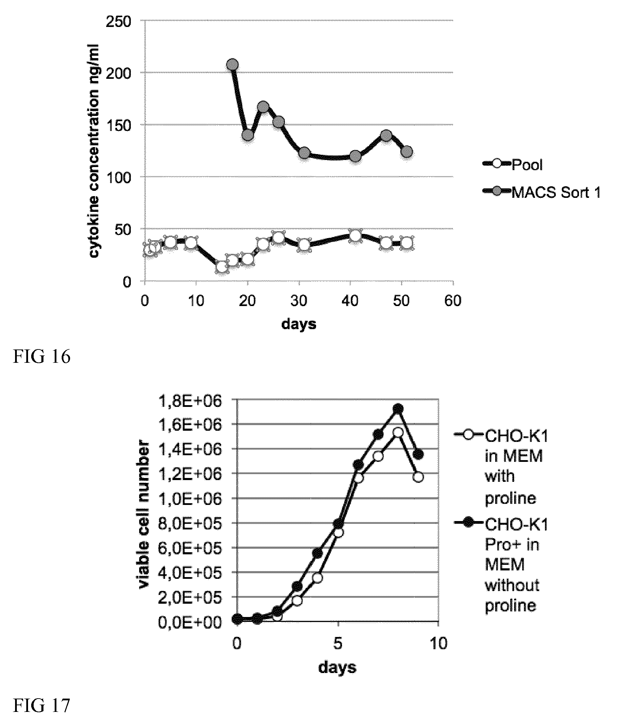

To verify the superiority of the MACS^{®} enrichment, again the process was in addition performed with a stable CHO-S cell line expressing a human cytokine as a second model protein and membrane-bound GFP as a reporter protein selected by the P5CS system (FIG 5, mGFP-P5CS I). The cell line generation is described in Example 2. As described above for the antibody-producing cell line the MACS^{®} enrichment was performed following the more protocol using to sequential MS columns, but using anti-GFP-MB (Miltenyi Biotec) for cell staining. The produced cytokine concentration could be enriched about 5-fold up to about over 200ng/ml (FIG 16 Pool to MACS Sort 1). The production decreased during the first week of culture, but stayed at a level of about 130ng/ml for 35 days after MACS^{®} Sort. The high productivity of the enriched cell population demonstrates the potency of MACS^{®} enrichment for an additional model protein.

### Example 5: Characterization in P5CS expression and selection in different CHO cell lines

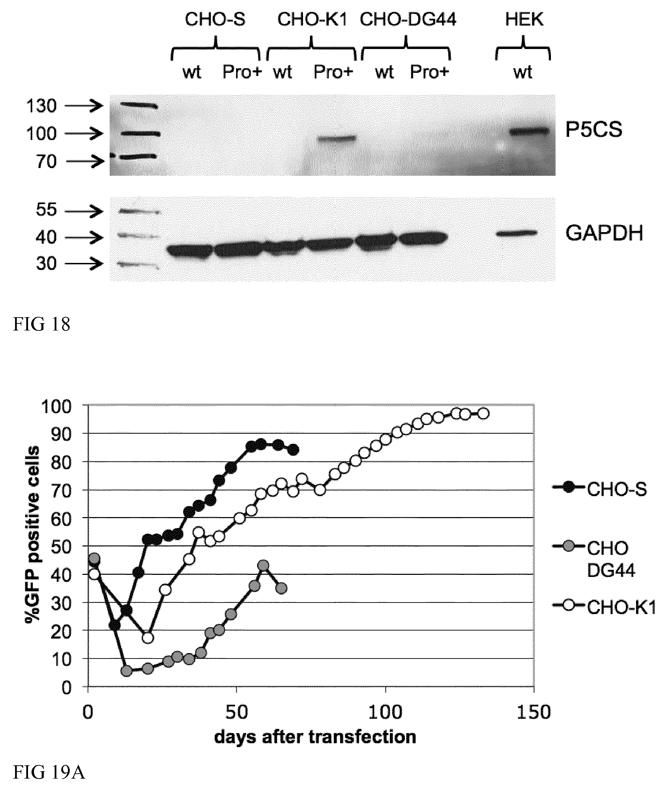

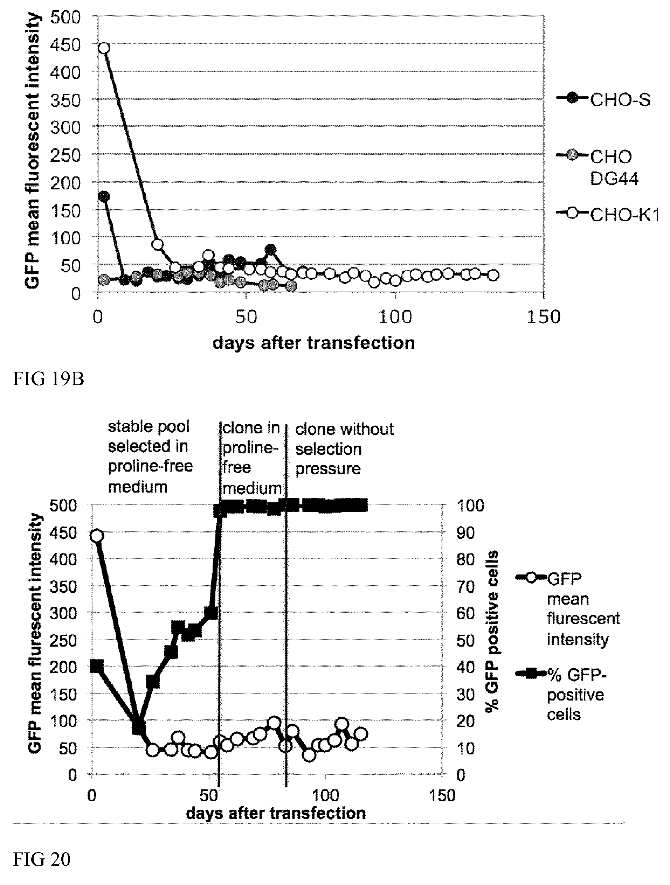

The cell lines CHO-K1 (ATCC, CCL-61^{™}), CHO-S (FreeStyle™ CHO-S Cells, Invitrogen, R800-07) and CHO DG44 (Invitrogen, A11000-01) wild-type cells were seeded in proline-free medium (MEM (Simga, M2279) supplemented with 2mM L-glutamine (PAA, M11-004), 10% dialyzed FBS (PAA, A15-507)). In all three cell lines the formation of prototroph colonies could be observed. For CHO-K1 the frequency of reversion to a prototroph phenotype is 0.01-0.015%; the growth of these Pro+ CHO-K1 in proline-free medium is comparable to the growth of CHO-K1 wild-type cells in medium containing proline (FIG 17). Western Blot analysis (Primary antibody: P5CS: anti-ALDH18A1 (Sigma, HPA012604); GAPDH: anti-GAPDH (abeam, ab9485); Secondary antibody: Stemgent HRP Mouse anti-Rabbit IgG Antibody (Miltenyi Biotec, 130-095-596)) of the CHO wild type and Pro+ cell reveal that no P5CS expression is detectable in CHO-S, CHO DG44 or CHO-K1 wild-type cells (wt). In the CHO-S Pro+ P5CS expression is not detectable, in difference a small amount of P5CS can be detected in CHO DG44 Pro+ and higher amounts in CHO-K1 Pro+ (FIG 18). As a positive control HEK cells (ATCC CRL-10852) were used.
To find the CHO cell line suited best for the P5CS selection system in addition all three CHO cell lines were transfected with a pMACS-CHO vector containing GFP as GOI and P5CS as selection marker as described in Example 1. 24 hours after transfection using Fugene HD transfection reagent (Roche) according to the manufacturer's instructions cells were expanded and selection pressure, medium without proline (MEM (Simga, M2279) supplemented with 2mM L-glutamine (PAA, M11-004), 10% dialyzed FBS (PAA, A15-507)), was applied. Selection media was changed once per week until stable cell lines grew up. Stable production cell lines were routinely passaged every 3-4 days in selection medium and expressing GFP monitored using MACSQuant^{®} Analyzer (Miltenyi Biotec). Under comparable high-serum selection conditions and analysis settings the stable CHO-S cell line has a GFP producer rate of 81% with a GFP mean fluorescent intensity of 54 at day 50 after transfection (FIG 19), followed by a GFP producer rate of 59% and GFP mean fluorescent intensity of 41 in CHO-K1 cells. CHO DG44 have the lowest producer rate and GFP mean fluorescent intensity at day 50, only 26% cell of the stable cell line produce GFP with a mean intensity of 18 (FIG 19). Clones from a GFP-expressing P5CS-selected CHO-K1 cell line were isolated by clone picking in semi-solid media. 50 cells per 6-well were seeded in selection medium. After attachment medium was changed with a semi-solid medium to avoid diffusion (2x MEM consisting of: 1:5 10x MEM Sigma, M0275, 1:17 Sodium Bicarbonate 7.5% Sigma S8761, 20% dialyzed FBS (PAA, A15-507), 4mM L-glutamine (PAA, M11-004); 2x MEM was mixed 1:1 with Genetix Clone Matrix, (Genetix, K8510)). 10 days after seeding the colonies were analysed by fluorescence microscopy. Bright green fluorescing colonies were marked and picked with a sterile pipette-tip into a 48-well. Clones were further expanded into T75, GFP fluorescence was analysed using the MACSQuant^{®} Analyzer (Miltenyi Biotec). Exemplary one clone is shown in FIG 20. The isolated clone has a GFP producer rate of 99% in medium containing no proline. The clone was also cultured in medium containing proline. Even in medium without selection pressure this isolated clone is expressing GFP with a stable producer rate and fluorescent intensity (FIG 20).

### Example 6: Recombinant antibody expression using a two-vector strategy with a combination of zeocin and P5CS selection

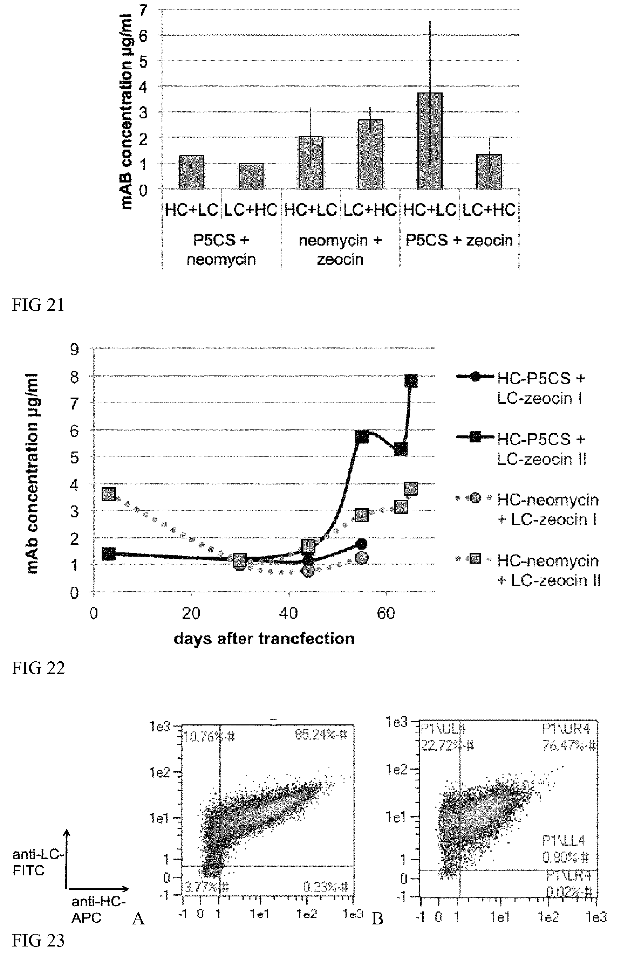

The cDNAs of heavy chain (HC) and light chain (LC) of a model antibody were cloned into two different tricistronic pMACS-CHO II vectors containing the P5CS gene and zeocin resistance gene (LC-zeocin resistance and HC-P5CS) or the neomycin resistance gene and zeocin resistance gene (LC-zeocin resistance and HC-neomycin resistance) as a selection marker. CHO-S (FreeStyle™ CHO-S Cells, Invitrogen, R800-07) cells were co-transfected using Fugene HD transfection reagent (Roche) according to the manufacturer's instructions. 24 hours after transfection cells were expanded and selection pressure, medium without proline containing appropriate concentrations of zeocin^{™} or neomycin (MEM (Simga, M2279) supplemented with 4mM L-glutamine (PAA, M11-004), 5% dialyzed FBS (PAA, A15-507), ITS+3 (Sigma, I2771) and 100/µg/mL zeocin^{™} (Invivogen, ant-zn-5) or 800µg/mL G418 (Merck, 345812)), or medium containing proline and appropriate concentrations of zeocin^{™} and neomycin (MEM (Simga, M2279) supplemented with 4mM L-glutamine (PAA, M11-004), 40mg/L proline (Sigma, P5607), 5% dialyzed FBS (PAA, A15-507), ITS+3 (Sigma, I2771), 100/µg/mL zeocin^{™} (Invivogen, ant-zn-5) and 800µg/mL G418 (Merck, 345812), was applied. Production levels were monitored by antibody concentration in cell culture supernatant at maximum cell densities by ELISA (coating antibody: IgG (Fc) (anti-human, clone JDC-10) Beckman Coulter 733164; detection antibody: anti-human kappa-HRP Miltenyi Biotec in-house). At day 55 after transfection stable cell lines generated by different selection marker combinations markers reveal that the combination of light chain - zeocin selection and heavy chain - P5CS selection leads to highest antibody concentrations with a maximum of 6.5 µg/ml (FIG 21).

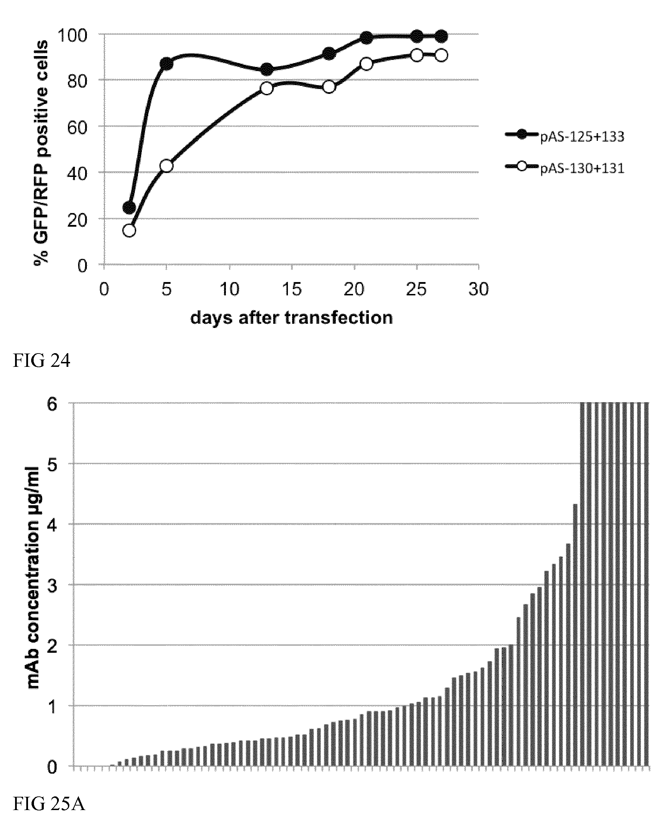

In further experiments the combination HC-P5CS selection combined with LC-zeocin selection was compared to a selection with two antibiotics (HC-neomycin selection combined with LC-zeocin selection). At day 55 after transfection the stable cell lines generated by zeocin and P5CS selection have produced antibody concentrations of 1.8 to 5.7µg/ml the stable cell lines generated by zeocin and neomycin selection have produced antibody concentrations of 1.2 to 2.8 µg/ml (FIG 22). Flow cytometry analysis using MACSQuant^{®} Analyzer (Miltenyi Biotec) of intracellular staining with the Inside Stain Kit (Miltenyi Biotec 130-090-447) of human kappa with a FITC labelled antibody (Miltenyi Biotec, Anti-Ig κ Light Chain-FITC, human, 130-093-053) and IgG with an APC labelled antibody (Miltenyi Biotec, Anti-IgG-APC, human, 130-093-194) according to manufacturer's instruction show producer rates of 85% for a combination of zeocin and P5CS selection and 76% for a combination of zeocin and neomycin selection at day 72 after transfection (FIG 23). Experiments with tricistronic vectors co-expressing GFP and RFP selected by a combination of zeocin and P5CS (LC-GFP-zeocin resistance gene and HC-RFP-P5CS= pAS-125+133 and LC-RFP-zeocin resistance gene and HC-GFP-P5CS= pAS-130+131, see FIG 24) reveal that producer rates of over 90% can be reached in less than 18 days monitored by percentage of GFP and RFP double-positive cells in flow cytometry analysis using MACSQuant^{®} Analyzer (Miltenyi Biotec).

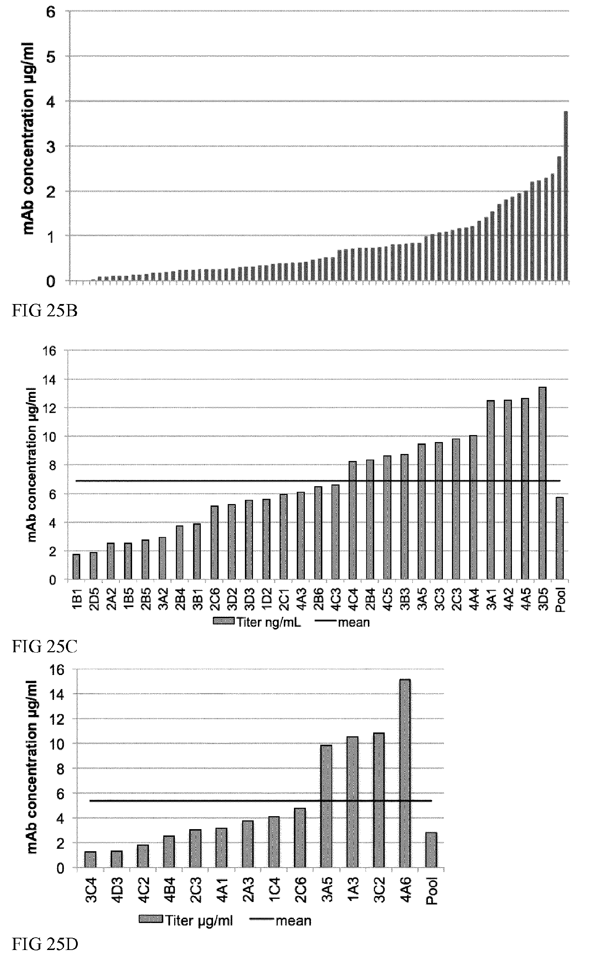

To further characterize the different selection marker combinations clones were isolated from highest producing cell lines from the bicistronic constructs (as described above zeocin and P5CS selection 5.7µg/ml, zeocin and neomycin selection 2.8µg/ml (FIG 22 and 23)) by limiting dilution as described in Example 3. Screening the supernatant of the 96-well plates for antibody production revealed that 15 of 81 clones (18.5 %) selected using zeocin and P5CS selection express more than 3µg/ml antibody (FIG 25 A) whereas only 1 of 75 clones (1.3 %) selected using zeocin and neomycin selection express more than 3µg/ml at similar cell densities (FIG 25 B).

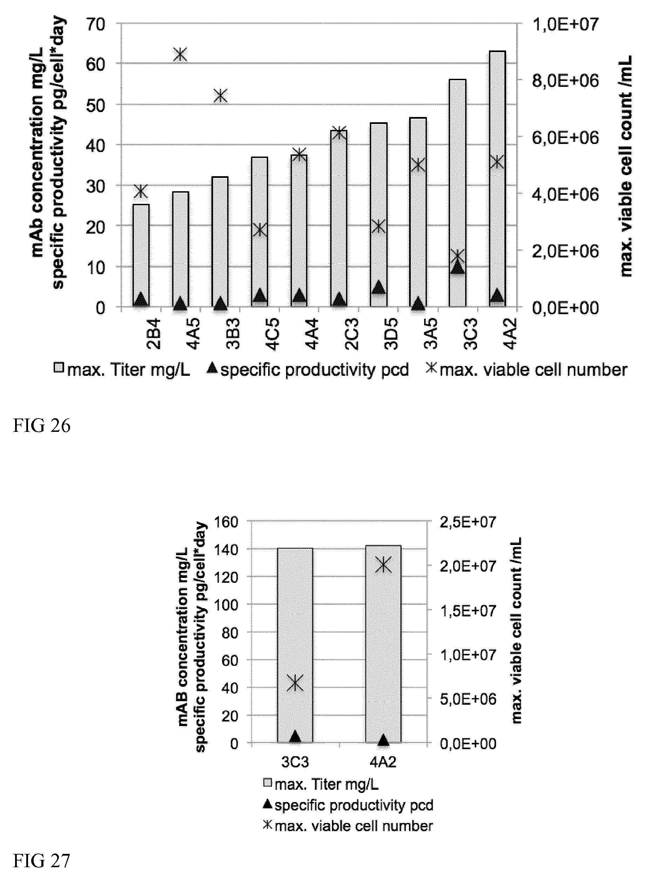

The best clones were expanded into T75 flasks. The mean produced antibody concentrations of all expanded clones was 6.9mµg/ml for clones from the zeocin and P5CS selection (FIG 25 C) combination and 5.4µg/ml for clones from the zeocin and neomycin selection combination (FIG 25 D). The 10 highest producing expanded clones from the zeocin and P5CS selection combination were adapted to growth in serum-free medium (CHO MACS CD without proline (custom made Miltenyi Biotec) containing 6 mM L-glutamine (PAA M11-004) and 100/µg/mL zeocin^{™} (Invivogen ant-zn-5)) and incubated in 125ml Erlenmeyer flasks in an orbital shake incubator at 125rpm in a batch process. Specific productivities of up to 10 (pg/(cell*day)) pg antibody production per cell per day and final antibody concentration in a batch process containing selection antibiotics of 63mg/L with maximal cell densities of 9*10⁶ viable cells /mL could be reached during this batch process (FIG 26). The two best clones (3C3 and 4A2) of the Batch process were cultivated in a Fed Batch process. Again 2*10⁵ cells /mL were seeded in in serum-free medium and cultivated as described above. Starting at day 3 cells were fed with CHO MACS Feed Supplement (Miltenyi Biotec, #170-007-003). For 2 days a feed of 3.1% of culture volume was given, 7.5% for another two days and finally 8% for the remaining culture time. Using a Fed Batch process for both clones a final antibody concentration of over 140 mg/mL in the supernatant could be reached. Clone 4A2 showed superior growth with a maximum living cell density of 2*10⁷ cells/mL; clone 3C3 showed lower viable cell densities of maximal 7*10⁶ cells /ml, but the higher specific productivity of 5 (µg/(cell*day)) pg antibody production per cell per day (FIG 27).

## Claims

1. A method for screening and enriching stable eukaryotic cells expressing high levels of a protein(s) of interest, the method comprising
a) transfecting or transducing eukaryotic host cells with at least one polynucleotide comprising a tricistronic expression cassette comprising
i) a promoter
ii) a gene of interest (GOI),
iii) a reporter gene,
iv) a selection marker gene,
v) an internal ribosome entry site (IRES) element
vi) a 2A element,
wherein the order in 5' to 3'direction within said tricistronic expression cassette is: promoter - GOI - IRES element operably linked to the reporter gene or the selection marker gene - 2A element operably linked to the reporter gene or the selection marker gene
b) culturing the eukaryotic host cells under conditions so as to express the protein(s) of interest, the reporter protein(s) and the selection marker protein(s) of said at least one polynucleotide in a cell culture selection medium for selecting positively-transfected/transduced cells by means of said selection marker protein(s)
c) screening, sorting and/or enriching the cells expressing high levels of protein(s) of interest by means of the reporter protein(s).

2. A method according to claim 1, wherein the screening, sorting and/or enriching of the cells is performed by magnetic cell sorting or flow cytometry sorting, and wherein at least one reporter protein is a membrane-bound reporter protein if magnetic cell sorting is used.

3. A method according to claim 1 or 2, wherein the screening, sorting and/or enriching of the cells is performed first by enrichment of the cells by magnetic cell sorting and subsequent second by sorting the cells by flow cytometry sorting.

4. A method according to any one of claims 1 to 3, wherein in said at least one polynucleotide the at least one reporter gene is a fluorescent gene.

5. A method according to claim 4, wherein at least one fluorescent gene of said at least one polynucleotide encodes for a membrane-bound fluorescent protein.

6. A method according to any one of claims 1 to 5, wherein one of the selection marker gene(s) is P5CS gene and wherein the eukaryotic cells are proline auxotroph.

7. A method according to any one of claims 1 to 6, wherein two or more of said polynucleotides are used, and wherein said polynucleotides are different in GOIs, reporter genes and selection marker genes.

8. A method according claim 7, wherein in a first polynucleotide the selection marker gene is P5CS gene and wherein in a second polynucleotide the selection marker gene is zeocin resistance gene, and wherein said eukaryotic cells are proline auxotroph and zeocin sensitive.

9. A method according to claims 7 or 8, wherein in said first polynucleotide the GOI is a gene encoding for a heavy chain of an antibody or a fragment thereof and wherein in said second polynucleotide the GOI is a gene encoding for a light chain of an antibody or a fragment thereof.

## Patentansprüche

1. Verfahren zum Untersuchen und Anreichern stabiler eukaryotischer Zellen, die hohe Werte eines/mehrerer relevanter Proteine exprimieren, wobei das Verfahren folgendes umfasst:
a) Transfizieren oder Transduzieren eukaryotischer Wirtszellen mit wenigstens einem Polynukleotid, umfassend eine trizistronische Expressionskassette, umfassend
i) einen Promotor,
ii) ein Gene of Interest (GOI),
iii) ein Reportergen,
iv) ein Selektionsmarker-Gen,
v) ein Element der internen ribosomalen Eintrittsstelle (IRES),
vi) ein 2A-Element,
wobei die Reihenfolge in 5'-zu-3'-Richtung in der trizistronischen Expressionskassette lautet: Promotor - GOI - IRES-Element, funktionsfähig verknüpft mit dem Reportergen oder dem Selektionsmarker-Gen - 2A-Element, funktionsfähig verknüpft mit dem Reportergen oder dem Selektionsmarker-Gen;
b) Kultivieren der eukaryotischen Wirtszellen unter Bedingungen, um das/die relevante(n) Protein(e), das/die Reporterprotein(e) und das/die Selektionsmarker-Protein(e) des wenigstens einen Polynukleotids in einem Zellkultur-Selektionsmedium zur Auswahl positiv transfizierter/transduzierter Zellen durch das/die Selektionsmarker-Protein(e) zu exprimieren;
c) Untersuchen, Sortieren und/oder Anreichern der Zellen, die hohe Werte des/der relevanten Proteins/Proteine durch das/die Reporterprotein(e) exprimieren.

2. Verfahren nach Anspruch 1, wobei das Untersuchen, Sortieren und/oder Anreichern der Zellen durch magnetische Zellsortierung oder Sortierung durch Durchflusszytometrie erfolgt, und wobei wenigstens ein Reporterprotein ein membrangebundenes Reporterprotein ist, wenn magnetische Zellsortierung verwendet wird.

3. Verfahren nach Anspruch 1 oder 2, wobei das Untersuchen, Sortieren und/oder Anreichern der Zellen erfolgt, indem zuerst die Zellen durch magnetische Zellsortierung angereichert werden und danach zweitens die Zellen durch Sortierung durch Durchflusszytometrie sortiert werden.

4. Verfahren nach einem der Ansprüche 1 bis 3, wobei in dem wenigstens einen Polynukleotid das wenigstens eine Reportergen ein fluoreszierendes Gen ist.

5. Verfahren nach Anspruch 4, wobei wenigstens ein fluoreszierendes Gen des wenigstens einen Polynukleotids für ein membrangebundenes fluoreszierendes Protein kodiert.

6. Verfahren nach einem der Ansprüche 1 bis 5, wobei eines der Selektionsmarker-Gene ein P5CS-Gen ist, und wobei die eukaryotischen Zellen prolinauxotroph sind.

7. Verfahren nach einem der Ansprüche 1 bis 6, wobei zwei oder mehr der Polynukleotide verwendet werden, und wobei sich die Polynukleotide in verschiedenen GOls, Reportergenen und Selektionsmarker-Genen befinden.

8. Verfahren nach Anspruch 7, wobei in einem ersten Polynukleotid das Selektionsmarker-Gen ein P5CS-Gen ist, und wobei in einem zweiten Polynukleotid das Selektionsmarker-Gen ein Zeocin-Resistenzgen ist, und wobei die eukaryotischen Zellen prolinauxotroph und empfindlich gegenüber Zeocin sind.

9. Verfahren nach Anspruch 7 oder 8, wobei in dem ersten Polynukleotid das GOI ein Gen ist, das für eine schwere Kette eines Antikörpers oder eines Fragments davon kodiert, und wobei in dem zweiten Polynukleotid das GOI ein Gen ist, das für eine leichte Kette eines Antikörpers oder eines Fragments davon kodiert.

## Revendications

1. Procédé de criblage et d'enrichissement de cellules eucaryotes stables exprimant des niveaux élevés d'une protéine(s) d'intérêt, le procédé comprenant :
a) la transfection ou la transduction de cellules hôtes eucaryotes avec au moins un polynucléotide comprenant une cassette d'expression tricistronique comprenant
i) un promoteur,
ii) un gène d'intérêt (GOI),
iii) un gène rapporteur,
iv) un gène marqueur de sélection,
v) un élément d'un site d'entrée de ribosome interne (IRES),
vi) un élément 2A,
dans lequel l'ordre dans la direction de 5' vers 3' au sein de la cassette d'expression tricistronique est : promoteur- GOI - élément IRES lié de façon fonctionnelle au gène rapporteur ou au gène marqueur de sélection
b) la culture de cellules hôtes eucaryotes dans des conditions telles de façon à exprimer la (les) protéines(s) d'intérêt, la (les) protéine(s) rapporteur et le(s) gène(s) marqueur(s) de sélection dudit au moins un polynucléotide dans un milieu de sélection pour la culture cellulaire destiné à la sélection de cellules positivement transfectées/transductées au moyen de ladite (desdites) protéine(s) marqueur de sélection
c) le criblage, le tri et/ou l'enrichissement des cellules exprimant des niveaux élevés de protéine(s) d'intérêt au moyen de la (des) protéine(s) rapporteur.

2. Procédé selon la revendication 1, dans lequel le criblage, le tri et/ou l'enrichissement des cellules est effectué par tri de cellules magnétiques ou par tri cytométrique continu, et dans lequel au moins une protéine rapporteur est une protéine rapporteur liée à une membrane si le tri de cellules magnétiques est utilisé.

3. Procédé selon la revendication 1 ou la revendication 2, dans lequel le criblage, le tri et/ou l'enrichissement des cellules est effectué premièrement par enrichissement des cellules par tri de cellules magnétiques et deuxièmement en triant ultérieurement les cellules par tri cytométrique continu.

4. Procédé selon l'une quelconque des revendications 1 à 3, dans lequel ledit au moins un polynucléotide l'au moins un gène rapporteur est un gène fluorescent.

5. Procédé selon la revendication 4, dans lequel au moins un gène fluorescent dudit au moins un polynucléotide code pour une protéine fluorescente liée à une membrane.

6. Procédé selon l'une quelconque des revendications 1 à 5, dans lequel un du (des) gène(s) marqueur(s) de sélection est le gène P5CS et dans lequel les cellules eucaryotes sont auxotrophes pour la proline.

7. Procédé selon l'une quelconque des revendications 1 à 6, dans lequel deux ou plus desdits polynucléotides sont utilisés, et dans lequel lesdits polynucléotides sont différents dans des GOIs, des gènes rapporteurs et des gènes marqueurs de sélection.

8. Procédé selon la revendication 7, dans lequel dans un premier polynucléotide le gène marqueur de sélection est le gène P5CS et dans lequel dans un second polynucléotide le gène marqueur de sélection est un gène résistant à la zéocine, et dans lequel lesdites cellules eucaryotes sont auxotrophes pour la proline et sensibles à la zéocine.

9. Procédé selon la revendication 7 ou la revendication 8, dans lequel dans ledit premier polynucléotide le GOI est un gène codant pour une chaine lourde d'un anticorps ou d'un fragment de celui-ci et dans lequel dans ledit second polynucléotide le GOI est un gène codant pour une chaine légère d'un anticorps ou d'un fragment de celui-ci.
